# EUROPEAN PATENT APPLICATION

(11) **EP 4 368 220 A1**
(43) Date of publication of application: **15.05.2024**
(21) Application number: 22206066.7
(22) Date of filing: 08.11.2022
(51) Int. Cl.: A61M 1/00, A61M 3/02

(54) **SYSTEM FOR FLUSHING A WOUND AND/OR A BODY CAVITY, IN PARTICULAR THE PERICARDIAL CAVITY**

(71) Applicant: Haermonics BV, 5616 LZ Eindhoven (NL)
(72) Inventor: Markus, Willem Paul, 5616 LZ Eindhoven (NL); Koch, Leonardus Jozef Lucas Maria, 5616 LZ Eindhoven (NL); Koolbergen, David Robert, 5616 LZ Eindhoven (NL)
(74) Representative: DTS Patent- und Rechtsanwälte Schnekenbühl und Partner mbB

(57) **Abstract**

The present invention relates to a flushing system (1, 300, 400, 500) configured to flush a wound and/or a body cavity, in particular the pericardial cavity (PC), of a patient, wherein the system (1, 300, 400, 500) comprises: an infusion liquid outlet (2) to connect to a first tube arrangement (4) having a plurality of one or more infusion liquid lumen (100, 100a, 100b, 100b) to guide a flow of infusion liquid from the system (1, 300, 400, 500) to the wound and/or the body cavity, in particular the pericardial cavity (PC); an effusion liquid inlet (6) to connect to a second tube (8) having an effusion liquid lumen (104) to guide the effusion liquid flow from the wound and/or the body cavity, in particular the pericardial cavity (PC), to the system (1, 300, 400, 500); a flow rate control system to control the flow rate of the infusion liquid flow at the infusion liquid outlet (2), wherein the flow rate control system comprises: a control unit (16) to provide one or more control signals, and a pump device (62) to pump infusion liquid to the infusion liquid outlet (2) at an infusion liquid flow rate, wherein the infusion liquid flow rate is adjustable by the control signals of the control unit (16), wherein each infusion liquid lumen (100, 100a, 100b, 100b) of the first tube arrangement (4) has at least one infusion liquid opening (102, 102a, 102b, 102b) for leading the infusion liquid into the wound and/or the body cavity, in particular the pericardial cavity (PC), wherein the infusion liquid openings (102, 102a, 102b, 102b) are arranged along the first tube arrangement (4) and are spaced from one another. The present invention further relates to a tube arrangement (4) for a flushing system (1, 300, 400, 500) configured to flush a wound and/or a body cavity, in particular the pericardial cavity (PC), of a patient.

## Description

The present invention relates to a flushing system configured for postoperative flushing of a wound and/or a body cavity, in particular the pericardial cavity, of a patient. In particular, the present invention relates to a flushing system for flushing a wound and/or a body cavity, in particular the pericardial cavity, of a patient as a postoperative treatment in order to reduce the risk of cardiac tamponade, reduce postoperative blood loss and reduce the accumulation of blood and clots in the wound and/or the body cavity, in particular the pericardial cavity.

The present invention further relates to a tube arrangement for a flushing system configured to flush a wound and/or a body cavity, in particular the pericardial cavity, of a patient, in particular for a flushing system as mentioned before.

At the end of a cardiac surgery procedure the pericardial sac is left open or partially closed, but will always have an open connection to the anterior mediastinum, which is the space behind the breastbone (sternum), between the lungs and in front of the heart. In further communication "the pericardial cavity" also refers to the anterior mediastinum while these cavities are in constant communication. The breastbone, which has been sawn and divided in two longitudinal parts, is an important source of blood loss postoperatively, but all the blood and clots accumulate around the heart, because of the posterior position (the patient lying on his back) and at the same time the anterior mediastinum is filled with blood and clots.

Excessive postoperative blood loss which might amount to more than 2 liters (2 I) per 24 hours or more than 200 ml per hour is a known complication of cardiac surgery and known to lead to other/more complications in cardiac surgery. Reoperation/re-exploration for bleeding is a strong independent risk factor for adverse outcome following cardiac surgery with higher mortality and morbidity rates. In addition, postoperative bleeding requiring multiple transfusions and surgical re-exploration is associated with higher costs, increased sternal wound infection, and transfusion-related infection.

To evacuate blood from the pericardial cavity - and if necessary pleural cavities - chest tubes are left postoperative. However, when blood loss is excessive or when clots start to develop more rapidly, the drains often fail in their function to evacuate all accumulated blood. Stasis of clots and blood in the pericardial (and/or pleural) cavity may lead to high fibrinolytic activity, maintenance of blood loss and in some cases to cardiac tamponade.

A pericardial flushing system may be specifically designed to clean the pericardial space after cardiac surgery. The system works by flushing the pericardial cavity with a saline (or other) solution, thereby lowering the viscosity and the hematocrit value of the blood present in the cavity and at the same time preventing the formation of larger clots. Cleaning the pericardial space by flushing will prevent clogging of the chest drains and the consequent accumulation of blood and clots in the pericardial space. This will lead to less postoperative bleeding and diminish the risk for provoking an acute cardiac tamponade.

In this respect, the system usually comprises tube means for flushing the pericardial cavity with the before-mentioned infusion liquid and for subsequently and/or simultaneously draining the pericardial cavity. However, as to the flushing and draining of the pericardial cavity, challenges may occur such as flushing the whole pericardial cavity and/or flushing the pericardial cavity in an even / uniform manner etc.

An example of prior art with respect to a pericardial flushing system can be found, e.g., in WO 2015/086857 A1.

Accordingly, it is an object of the invention to provide a flushing system configured to flush a wound and/or a body cavity, in particular the pericardial cavity, of a patient and having enhanced flushing properties, in particular, with respect to effectiveness and/or efficiency.

This is achieved by a flushing system configured to flush a wound and/or a body cavity, in particular the pericardial cavity, of a patient as defined in claim 1.

Correspondingly, a flushing system configured to flush a wound and/or a body cavity, in particular the pericardial cavity, of a patient is provided, wherein the system comprises:
- an infusion liquid outlet to connect to a first tube arrangement having one or more (e.g., a plurality of) infusion liquid lumen to guide a flow of infusion liquid from the system to the wound and/or the body cavity, in particular the pericardial cavity,
- an effusion liquid inlet to connect to a second tube having an effusion liquid lumen to guide the effusion liquid flow from the wound and/or the body cavity, in particular the pericardial cavity (e.g., and/or the pleural cavity, in particular, the left and/or right pleural cavity), to the system,
- a flow rate control system to control the flow rate of the infusion liquid flow at the infusion liquid outlet, wherein the flow rate control system comprises:
   - a control unit to provide one or more control signals, and
   - a pump device to pump infusion liquid to the infusion liquid outlet at an infusion liquid flow rate, wherein the infusion liquid flow rate is adjustable by the control signals of the control unit,
wherein each infusion liquid lumen of the first tube arrangement has at least one infusion liquid opening for leading the infusion liquid into the wound and/or the body cavity, in particular the pericardial cavity, wherein the infusion liquid openings are arranged along the first tube arrangement and are spaced from one another.

The invention is particularly based on the idea that, due to the infusion liquid lumens of the first tube arrangement and due to the arrangement of their corresponding openings along the first tube arrangement, the wound and/or the body cavity, in particular the pericardial cavity, can be flushed in a more effective manner. In particular, it may facilitate to flush everything, i.e., everything being intended to be flushed (e.g., blood and/or clots), from the wound and/or the body cavity, in particular the pericardial cavity, due to the multi-lumen and its distribution of corresponding openings. In other words, the flushing liquid may be intended to reach as much as possible of the areas of the space of the wound and/or the body cavity, in particular between the heart and the pericardium, without forming channels between the points of inflow and outflow. Further, since the infusion liquid lumens may also allow guiding a predetermined inflow of infusion liquid through each lumen and corresponding opening/openings, the wound and/or the body cavity, in particular the pericardial cavity, can also be flushed in a more efficient manner. In respect to the above, the distribution of openings along the first tube arrangement may be considered as a rainforest-shower-like arrangement of openings, by means of which the distribution of flushing liquid, i.e., infusion liquid, may take place in a more uniform and smoother manner, thereby facilitating an enhanced flushing of the wound and/or the body cavity, in particular the pericardial cavity.

As mentioned above, the first tube arrangement has one or more (e.g., a plurality of) infusion liquid lumen to guide a flow of infusion liquid from the system to the wound and/or the body cavity, in particular the pericardial cavity. In this respect, one or more infusion liquid lumen may comprise one infusion liquid lumen, or may comprise at least two infusion liquid lumens, or may comprise two or more infusion liquid lumens (for example, only two infusion liquid lumens).

As mentioned above, each infusion liquid lumen of the first tube arrangement has at least one infusion liquid opening for leading the infusion liquid into the wound and/or the body cavity, in particular the pericardial cavity. In this respect, at least one infusion liquid opening may comprise only one infusion liquid opening, or may comprise one or more infusion liquid openings.

The first tube arrangement may include a retractable sheath being retractable between a first position and a second position via at least one intermediate position, along the first tube arrangement, wherein, in the first position, the infusion liquid openings may be covered by the sheath, in the second position, the infusion liquid openings may not be covered by the sheath, and, in the intermediate position, some of the infusion liquid openings may be covered by the sheath.

The sheath may be configured to extend along the first tube arrangement. The sheath may further be configured to cover at least a part of the first tube arrangement, to thereby allow that, in the first position, the infusion liquid openings may be covered by the sheath, in the second position, the infusion liquid openings may not be covered by the sheath, and, in the intermediate position, some of the infusion liquid openings may be covered by the sheath.

Means for reversibly retracting the sheath may be assigned to the sheath. Hence, said means may be included in the flushing system. In this respect, any suitable means may be conceivable, such as a mechanical means for pushing and/or pulling the sheath, to thereby move the sheath between the above-mentioned positions. Said means may be positioned inside or in connection with the first tube arrangement and/or the second tube.

The first tube arrangement may be arrangeable in a substantially horizontal orientation for leading the infusion liquid into the wound and/or the body cavity, in particular the pericardial cavity. Accordingly, the infusion liquid may be uniformly and smoothly guided into the wound and/or the body cavity, in particular the pericardial cavity. Further, the rainforest-shower-like delivery of infusion liquid may thereby be provided.

The effusion liquid lumen of the second tube may have at least one effusion liquid opening for receiving the effusion liquid from the wound and/or the body cavity, in particular the pericardial cavity. In other words, the effusion liquid lumen may comprise one or more effusion liquid openings for receiving the effusion liquid from the wound and/or the body cavity, in particular the pericardial cavity. The effusion liquid opening(s) may be arranged along the second tube and may be spaced from one another. The effusion liquid openings may be larger (e.g., with respect to a footprint area and/or a diameter) than the infusion liquid openings, respectively. Further, since clots are reduced and/or minimized, or do no longer occur, the overall diameter in this regard may advantageously be reduced, i.e., may advantageously become smaller.

The first tube arrangement and the second tube may be configured to be arranged or arrangeable such that the effusion liquid openings and the infusion liquid openings are disposed at different levels with respect to the gravitational direction for allowing the infusion liquid to either work with or against gravity, when applied in the wound and/or the body cavity, in particular the pericardial cavity. Hence, the gravitational force may either support the flushing of the wound and/or the body cavity, in particular the pericardial cavity, in case of providing the liquid at a higher level than draining the liquid, or may allow for a more uniform and smoother flushing of the wound and/or the body cavity, in particular the pericardial cavity, in case of providing the liquid at a lower level than draining the liquid.

In particular, due to the multiple inflow points of the first tube arrangement, irrigation channel formation may advantageously be prevented and/or the flushing of the whole wound and/or the whole body cavity, in particular the whole pericardial cavity, may advantageously be optimized and/or achieved.

The first tube arrangement and the second tube may be combined in an integrated inflow and/or outflow drain device. In this case, the infusion liquid outlet and the effusion liquid inlet may also be combined in an integrated liquid interface device being configured to connect to the integrated inflow and/or outflow drain device. For example, the first tube arrangement may be integrated in the second tube in such a way that the first tube arrangement does not consume lumen of the second tube. In particular, the first tube arrangement may be positioned in the wall of the second tube. It is preferred that the integrated inflow and/or outflow drain device should have a regular shape, such as a rounded or circular shape, in order to be placeable or placed in the patient's body without any leakage from the wound and/or the body cavity, in particular the pericardial cavity (and/or the pleural cavity).

The integrated first tube arrangement of the integrated inflow and/or outflow drain device may be longer than the integrated second tube.

The integrated first tube arrangement may have a length such as to allow that the effusion liquid openings and the infusion liquid openings are arranged or arrangeable at different levels with respect to the gravitational direction. Additionally or alternatively, the integrated second tube may have a length such as to allow that the effusion liquid openings and the infusion liquid openings are arranged or arrangeable at different levels with respect to the gravitational direction. In other words, the length of the integrated first tube arrangement may either be longer or shorter than the integrated second tube, such as to allow that the effusion liquid openings and the infusion liquid openings are arranged or arrangeable at different levels with respect to the gravitational direction. In still other words, alternatively, the integrated second tube of the integrated inflow and/or outflow drain device may be longer than the integrated first tube arrangement.

In this respect, different lengths of the (e.g., integrated) first tube arrangement and the (e.g., integrated) second tube may serve for positioning the effusion liquid openings and the infusion liquid openings at some distance from each other to prevent the inflow liquid from leaving the wound and/or the body cavity, in particular the pericardial cavity, immediately by the shortest pathway out of the wound and/or the body cavity, in particular the pericardial cavity, through the outflow tube (e.g., the second tube) and thereby not flushing the wound and/or the body cavity, in particular the pericardial cavity.

The system may further comprise one or more effusion liquid containers to receive effusion liquid from the wound and/or the body cavity, in particular the pericardial cavity. The one or more effusion liquid containers may be connectable or connected to the effusion liquid inlet for receiving effusion liquid from the wound and/or the body cavity, in particular the pericardial cavity.

The flushing system may be configured to provide approximately 50-2000 ml of infusion liquid per hour, e.g., approximately 500 ml of infusion liquid per hour, i.e., the infusion inflow may be approximately 50-2000 ml per hour, e.g., approximately 500 ml of infusion liquid per hour, wherein, optionally, this may depend on the patient and/or clinical outcome, bleeding and/or other medical / clinical parameters. However, the system and, accordingly, the one or more effusion liquid containers may be configured to handle sudden gushes of 400 ml and/or 500 ml of effusion liquid per 5 min.

The system may further comprise a hematocrit sensor configured to measure the hematocrit value of the effusion liquid for obtaining a blood loss volume and/or a blood loss flow rate from the wound and/or the body cavity, in particular the pericardial cavity.

The hematocrit sensor may be configured to optically measure the hematocrit value of the effusion liquid, which may be receivable or received via the effusion liquid inlet, for obtaining a blood loss volume and/or a blood loss flow rate from the wound and/or the body cavity, in particular the pericardial cavity.

The hematocrit sensor may be disposed between the effusion liquid inlet and one or more effusion liquid containers for conducting measurements in the effusion liquid. In other words, the hematocrit sensor may be arranged downstream from the effusion liquid inlet and upstream from the one or more effusion containers for conducting measurements in the effusion liquid.

The flushing system may further comprise one or more suction devices to draw effusion liquid out of the wound and/or the body cavity, in particular the pericardial cavity. The one or more suction devices may be arranged to create a relative low pressure in the one or more effusion liquid containers to receive effusion liquid so that effusion liquid is drawn towards the one or more effusion liquid containers. For example, the one or more effusion liquid containers may be connectable or connected to an intensive care unit (ICU) vacuum wall connector or the like, wherein the ICU vacuum wall connector may be configured to create said (relative) low pressure and may thereby act as said one or more suction devices. For example, the one or more suction devices may be integrated in the system, to thereby provide a mobile and/or independent solution, in particular being independent in view of special vacuum range requirements not provided by the hospital wall connectors.

The pump device may be any suitable pump device for infusion liquid, and may preferably be of a type that allows pumping at a relative accurate flow rate. In this respect, the pump device may be a peristaltic pump. It may additionally or alternatively be conceivable, that the pump device may comprise a volumetric pump, a membrane pump, an impeller pump and/or a syringe pump device including a syringe pump and, optionally, at least two valves. The pump device may be connectable or connected to one or more infusion liquid containers.

Hence, optionally, the system may comprise above-mentioned one or more infusion liquid containers containing infusion liquid, for example a number of bags containing saline fluid, such as NaCI 0,9%, as known in the art, e.g., clinical practice. The one or more infusion containers may be connectable or connected to the pump device being configured to pump the infusion liquid with a desired flow rate towards the infusion liquid outlet.

The infusion liquid flow rate of the pump device may be adjustable by the control unit so that the control unit may control the flow rate of infusion liquid that is pumped to the wound and/or the body cavity, in particular the pericardial cavity.

The system may further comprise a pressure sensor being positioned inside or in connection with the first tube arrangement, the second tube or the wound and/or the body cavity, in particular the pericardial cavity, and being configured for obtaining the pressure in the wound and/or the body cavity, in particular the pericardial cavity. In other words, the pressure sensor may be configured to provide a pressure signal representative for the pressure in the wound and/or the body cavity, in particular the pericardial cavity. The obtained pressure may be provided to the control unit and/or a pressure control unit, which may be part of the control unit or a separate unit of the system and which may be configured to provide a warning and/or an alarm and/or a message to a user, e.g., clinical staff, and/or to adjust the infusion liquid flow rate to keep the pressure (i.e., for example, the pressure signal) within desired pressure limits so as to avoid acute cardiac tamponade. It may also be conceivable that the system may further comprise a pressure sensor being positioned inside or in connection with the first tube arrangement, in other words inside or in connection with an infusion lumen (e.g., at least one of the one or more infusion liquid lumen), for obtaining the pressure in the wound and/or the body cavity, in particular the pericardial cavity. In other words, the system may be configured for measuring pressure in the wound and/or the body cavity, in particular the pericardial cavity, over or via an/the infusion lumen (e.g., at least one of the one or more infusion liquid lumen).

The pressure sensor may be integrated in the integrated inflow and/or outflow drain device for measuring an exact pressure (in the wound and/or the body cavity, in particular the pericardial cavity) at the proximal end of the integrated inflow and/or outflow drain device, the first tube arrangement and/or the second tube.

The system may further comprise a heater device configured to heat the infusion liquid being intended to be guided to the wound and/or the body cavity, in particular the pericardial cavity, to a desired infusion liquid temperature.

The heater device may be part of a temperature control system of the system, which may be configured to control a temperature of the infusion liquid flow, wherein the temperature control system may accordingly comprise: a temperature sensor to measure a temperature of infusion liquid, a temperature control unit, which may be part of the control unit or a separate unit of the system and which may be configured to provide a temperature control signal on the basis of the measured temperature of infusion liquid and a desired infusion liquid temperature, and said heater device, wherein the heater device may be controlled / adjusted by the temperature control signal to heat the infusion liquid to the desired infusion liquid temperature. The temperature sensor may be arranged in or on the first tube arrangement and/or the second tube, or in or on the integrated inflow and/or outflow drain device. The temperature sensor may also be configured as or in a separate element (e.g., for rectal application) being connected or connectable to the control unit and/or the temperature control unit. The temperature sensor may be integrated in the integrated inflow and/or outflow drain device in order to provide an exact measurement of a central body temperature.

The desired infusion liquid temperature may be based on a temperature of the wound and/or the body cavity, in particular the pericardial cavity, in particular a desired temperature of the wound and/or the body cavity, in particular the pericardial cavity (PC), and/or a general body temperature. Preferably, the infusion liquid temperature may be in a range of 36°C and 38°C, more preferably approximately 37°C at the moment the liquid reaches the body. The desired infusion liquid temperature may also be adapted or adjusted to the actual body temperature of the patient.

Additionally or alternatively, the infusion liquid, i.e., the inflow liquid, may, e.g., if the clinical situation demands, be used to cool down the patient, e.g., the wound and/or the body cavity, in particular the pericardial cavity. For instance, in case the patient is in a "cooling protocol" after a serious adverse event with total circulatory collapse, the patient may be cooled down to a range of 34°C and35°C, in particular in order to diminish ischaemic damage to organs. In current state of art, patients are cooled down with external cooling elements around the legs and arms. Cooling down internally with the flushing system may help, especially while the goal may be reducing a/the central body temperature. For example, the heart itself may need to be cooled down in case of certain rhythm disturbances.

The system may further comprise at least one further effusion liquid inlet to connect to at least one further tube having an effusion liquid lumen to guide the effusion liquid flow from a (e.g., further) wound and/or a (e.g., further) body cavity, in particular the pleural cavity or the pericardial cavity, to the system. For example, the system may further comprise a further effusion liquid inlet to connect to a third tube having an effusion liquid lumen to guide the effusion liquid flow from the pleural cavity to the system. Accordingly, the at least one further effusion liquid inlet may be referred to as a second effusion liquid inlet, a third effusion liquid inlet etc. depending on the number of effusion liquid inlets, wherein the effusion liquid inlet may then be accordingly referred to as the first effusion liquid inlet. As to the tubes, the at least one further tube may be referred to as a third tube assigned to the second effusion liquid inlet, a fourth tube assigned to the third effusion liquid inlet etc. For example, the system may further comprise a second effusion liquid inlet to connect to a third tube having an effusion liquid lumen to (e.g., additionally) guide the effusion liquid flow from the wound and/or the body cavity, in particular the pericardial cavity, to the system. The effusion liquid flows of the second and the third tubes may be merged by means of a fluid connector piece, such as a Y-shaped connector piece, in order to allow the liquid to be subsequently guided towards the hematocrit sensor. Alternatively or additionally, the system may further comprise a third effusion liquid inlet to connect to a fourth tube having an effusion liquid lumen to guide the effusion liquid flow from the wound and/or the body cavity, in particular the pleural cavity, to the system. In other words, the system may further comprise a further, e.g., third, effusion liquid inlet to connect to a further, e.g., fourth, tube having an effusion liquid lumen to guide the effusion liquid flow from the wound and/or the body cavity, in particular the pleural cavity, to the system. Optionally, the system may further comprise a fourth effusion liquid inlet to connect to a fifth tube having an effusion liquid lumen to guide the effusion liquid flow from the wound and/or the body cavity, in particular the pleural cavity, to the system. The effusion liquid flows of the fourth and the fifth tubes may be merged by means of a fluid connector piece, such as a Y-shaped connector piece, in order to allow the liquid to be subsequently guided towards the hematocrit sensor or towards a further hematocrit sensor (to be described further below) being particularly assigned to the liquid being received from the wound and/or the body cavity, in particular the pleural cavity.

The system may further comprise a further hematocrit sensor configured to measure the hematocrit value of the effusion liquid from the wound and/or the body cavity, in particular the pleural cavity, for obtaining a blood loss volume and/or a blood loss flow rate from the wound and/or the body cavity, in particular the pleural cavity.

The further hematocrit sensor may be configured in the same way as the hematocrit sensor as described herein, in particular, as previously described as well as in the following. In this respect, the further hematocrit sensor may be referred to as a/the second hematocrit sensor, and the hematocrit sensor may be referred to as a/the first hematocrit sensor.

When the system is configured to also receive effusion liquid from the wound and/or the body cavity, in particular the pleural cavity, the second hematocrit sensor may be arranged in a parallel effusion liquid line assigned to the effusion liquid received from the wound and/or the body cavity, in particular the pleural cavity, and may be correspondingly arranged in the same manner as outlined above with respect to the (first) hematocrit sensor. Hence, the further hematocrit sensor may be disposed between the further effusion liquid inlet and one or more effusion liquid containers for conducting measurements in the effusion liquid.

The system may further comprise at least one means (e.g., sensor) for obtaining an effusion liquid volume and/or an effusion liquid flow rate of the effusion liquid received from the wound/wounds and/or the body cavity/cavities, in particular the pericardial cavity and/or the pleural cavity. In particular, the system may further comprise at least one means (e.g., sensor) for obtaining an effusion liquid volume and/or an effusion liquid flow rate of the effusion liquid received from the wound/wounds and/or the body cavity/cavities, in particular the pericardial cavity and/or the pleural cavity, and guided through the hematocrit sensor and/or the further hematocrit sensor.

Furthermore, the system can further comprise at least one weight sensor, which is to measure a change in weight of the infusion liquid in at least a part and/or component of the system, especially in infusion liquid container of the system. The change in weight can be used as a representative value for a decrease in the volume of infusion liquid in the system, especially in the infusion liquid container of the system. On the basis of the change of volume of infusion liquid over the course of time, the flow rate of infusion liquid towards the pericardial cavity PC may be determined.

Additionally or alternatively the system may further comprise a buffer container having a buffer volume and comprising a buffer inlet to receive effusion liquid from the wound and/or the body cavity, in particular the pericardial cavity, and a first buffer outlet to output the received effusion liquid.

The buffer container may comprise an (optional) clot trap configured for preventing clots included in the effusion liquid being received from the wound and/or the body cavity, in particular the pericardial cavity, from exiting the buffer container via the first buffer outlet, and/or wherein the buffer container is configured for air-liquid-separation, and/or configured with a safety bypass, and/or configured for receiving liquid gushes.

The basic idea of the buffer container may be that the buffer container may provide different functions, which may dependently or independently be fulfilled and/or occur, wherein, firstly, clots may be trapped by said clot trap, secondly, air and liquid may be separated from each other, thirdly, sudden gushes of effusion liquid may be collected, and, fourthly, effusion liquid may be provided for discreet measurements. Further, due to air-liquid-separation and/or the safety bypass, a constant vacuum may be applicable to the wound and/or the body cavity, in particular the pericardial cavity, for draining. In this respect, on the one hand, the effusion liquid is buffered before being guided further for subsequent proceedings (e.g., analysis) and, on the other hand, the effusion liquid is freed from clots. This may be achieved by providing a/the buffer container comprising a/the clot trap. The clot trap prevents clots included in the effusion liquid from being guided further. The clot trap may be a mechanical means. Due to buffering of the effusion liquid, a more steady flow with a certain (e.g., known) volume leaving the buffer container for further proceedings may be achieved, to thereby allow subsequent sensor means to conduct more precise measurements. Additionally, due to the removal of the clots, subsequent sensor means may also be allowed to conduct more precise measurements. Furthermore, the buffer container may serve as a safety means with respect to pressure. In this respect, the buffer volume of the buffer container may store liquid which might (otherwise) have caused back pressure / afflux in the system (e.g., towards the effusion liquid inlet). Further, the buffer container may vent the effusion liquid such that air may be discharged therefrom in order not to influence a subsequent analysis / measurements. In other words, the buffer container may be configured to separate air from liquid, in particular the effusion liquid.

The buffer container may substantially have a cylindrical shape extending along a central axis. However, it should be understood that any other shape may be conceivable in this regard. The buffer container may comprise a top end and a bottom end. The buffer container may comprise a top wall, a side wall and a bottom wall. The buffer inlet may be disposed closer to the top wall than to the bottom wall. The first buffer outlet may be disposed closer to the bottom wall than to the top wall. The buffer inlet may be arranged in the top wall or the side wall. The first buffer outlet may be arranged in the bottom wall or the side wall. Hence, the buffer inlet may be arranged at a higher level than the first buffer outlet with respect to the gravitational direction. When the buffer container is incorporated, e.g., mounted, in the system, the central axis substantially extends along the gravitational direction.

Since the flushing system may be configured to provide approximately 500 ml of infusion liquid per hour, i.e., the infusion inflow may be approximately 500 ml per hour, the buffer volume may be sized accordingly. However, the system and, accordingly, the buffer volume may be configured to handle sudden gushes of 400 ml and/or 500 ml of effusion liquid per 5 min.

The clot trap may comprise a sieve structure being disposed between the buffer inlet and the first buffer outlet of the buffer container, to thereby prevent clots from passing through the first buffer outlet. The clot trap may be integrally formed with the buffer container (e.g., on the side wall or the bottom wall of the buffer container). Alternatively, the clot trap may be mounted to the buffer container via fixing means. The sieve structure may comprise a plurality of openings formed therein. The openings may be configured to stop clots from passing through the sieve structure.

The clot trap may have a conical shape having a base facing towards the first buffer outlet and an apex facing the buffer inlet. It is also possible, that the clot trap may have other shapes, e.g., a truncated cone shape, a dome shape, a flat shape etc. In particular, due to said shape of the clot trap, certain amounts of clots may be trapped in the clot trap without blocking the clot trap.

The buffer container may further comprise at least one level sensor for obtaining at least one filling level of the buffer volume.

The buffer container may further comprise a first level sensor and a second level sensor for obtaining a first filling level and a second filling level of the buffer volume, respectively, wherein the second level sensor may be arranged at a higher level than the first level sensor with respect to the gravitational direction.

A level sensor (e.g., the above level sensors) may be configured to obtain a (the) filling level, for example, mechanically or optically. However, any other suitable sensor may also be applicable (such as capacitive sensors, resistive sensors, ultrasonic sensors etc.).

The control unit may provide at least one of the control signals on the basis of the obtained filling level of the buffer volume and/or a buffer container level, to thereby control the first pump device and adjust the effusion liquid rate (e.g., pumped volume) from the buffer container. Hence, the buffer container may collect effusion liquid until the first pump device is allowed to pump. Accordingly, a steady flow of effusion liquid may be generated in a controlled manner. As a consequence, subsequent analysis means may perform precise measurements in/at the effusion liquid. The control unit may be configured to control the first pump device in order to pump a predetermined, i.e., a known, volume on the basis of said control signals. For example, the control unit may be configured to control the first pump device in order to pump a volume of, e.g., 30 ml, which may be referred to as a batch. However, any other volume may be pumped on the basis of the control signals of the control unit. Accordingly, the first pump device may be operated in a discontinuous manner by the control unit. Additionally or alternatively, the first pump device may be controlled by a control signal being based on an amount of outflow liquid.

The system may further comprise the (above-mentioned) one or more effusion liquid containers to receive effusion liquid out of the buffer container. The one or more effusion liquid containers may be connectable or connected to the buffer container (e.g., via the first buffer outlet).

The buffer container may further comprise a second buffer outlet being arranged at a higher level than the first buffer outlet with respect to the gravitational direction and being configured to output effusion liquid and/or air, to thereby allow an air-liquid-separation and/or allow to function as the safety bypass. Further, the second buffer outlet may be arranged at a lower level than the buffer inlet with respect to the gravitational direction. The second buffer outlet may be connectable or connected to the one or more effusion liquid containers. For example, the second buffer outlet may be connectable or connected to the one or more effusion liquid containers in a direct and/or an always open manner.

The second buffer outlet may be disposed on an opposite side of the clot trap with respect to the first buffer outlet. Hence, the second buffer outlet may be disposed on the same side as the buffer inlet.

The second buffer outlet may act as a safety overflow and/or a safety bypass from the buffer container, to thereby provide an open connection to the one or more effusion liquid containers for safety reasons (e.g., for preventing back pressure /afflux). Further, the second buffer outlet may also provide an exit path for air being separated from the effusion liquid in the buffer container. The separated air may be sucked into the one or more suction devices, e.g., the ICU vacuum wall connector and/or a system's own vacuum unit. In other words, the separated air may be sucked via the one or more effusion liquid containers into the ICU vacuum wall connector and/or into the system's own vacuum unit. Hence, due to the lack of air in the effusion liquid, the effusion liquid may subsequently be analyzable or analyzed in a more accurate and reliable manner.

The hematocrit sensor may be disposed for conducting measurements in the effusion liquid pumped from the buffer container to the one or more effusion liquid containers. Hence, the hematocrit sensor may be arranged downstream from the buffer container and upstream from the one or more effusion containers. Due to said arrangement of the hematocrit sensor, precise hematocrit measurements may be conducted without negative effects of clots and/or air in the effusion liquid. Further, since the first pump device may be controlled with respect to the volume of liquid to be pumped by the control unit, the measured hematocrit value may be set in correlation with the pumped volume, in which the hematocrit value is measured by said sensor. Hence, the blood loss volume and/or the blood loss flow rate from the wound and/or the body cavity, in particular the pericardial cavity, may be measured in an easy, reliable and/or accurate manner.

Additionally or alternatively, the pressure sensor may be positioned inside or in connection with the buffer container.

When the system is configured to also receive effusion liquid from the pleural cavity, the system may also comprise a further buffer container (i.e., a second buffer container), a further pump device (i.e., a third pump device), and a further hematocrit sensor (i.e., a second hematocrit sensor), wherein each of said further elements of the system are configured as outlined above with respect to the buffer container, the first pump device and the hematocrit sensor. The second buffer container, the third pump device and the second hematocrit sensor may be arranged in a parallel effusion liquid line, for example a parallel effusion liquid measurement line, assigned to the effusion liquid received from the pleural cavity and may be correspondingly arranged in the same manner as outlined above with respect to the buffer container, the first pump device and the hematocrit sensor. It may be conceivable, that this secondary system (buffer container, pump and hematocrit sensor) may be configured and/or used to separately measure the blood loss and/or air leakage.

It may be conceivable that the buffer container (and/or the second buffer container) may be configured as a cartridge or as a part of a cartridge and/or a frame construction. Optionally, the cartridge or the frame construction is a disposable, in particular a disposable cartridge or a disposable frame construction. It may be conceivable, that the cartridge or the frame construction may be provided with interface elements for receiving and/or transmitting liquid and/or signals, such as control signals and/or sensor signals, from and/or to the system or a part of the system. Further, at least one of the interface elements may be configured for a power supply. Correspondingly, the system may be provided with counter interface elements corresponding to and being engageable with the interface elements of the cartridge. It may additionally or alternatively be conceivable, that the frame construction may hold or comprise one or more tubes for connecting to the counter interface elements, e.g., for connecting with pump devices, sensor devices and/or valve devices of the system.

In other words, a cartridge or frame construction, in particular a disposable cartridge or frame construction, may be provided, which may be configured to interface with the system and/or a part of the system and which may comprise the buffer container (and/or the second buffer container). Accordingly, the flushing system may be provided as described above, wherein the buffer container may be substituted with the cartridge or frame construction.

In a further embodiment, the cartridge or frame construction may also comprise the first pump device (and/or the third pump device, when the cartridge comprises the second buffer container) and/or may be engageable therewith. In a still further embodiment, the cartridge may also comprise the first pump device and the hematocrit sensor (and/or the third pump device and the second hematocrit sensor, when the cartridge comprises the second buffer container) and/or may be engageable therewith. In a still further embodiment, the cartridge or frame construction may additionally comprise the pressure sensor and/or interface and/or membrane to the pressure sensor on the belonging device. In a still further embodiment, the cartridge or frame construction may additionally comprise a lumen element assigned to the heater device and configured for guiding infusion liquid through the heater device for tempering the infusion liquid. It should be understood that connecting elements and connecting lines between the above-described elements for guiding infusion and/or effusion liquid may also be included in or on the cartridge or frame construction and may accordingly be part of the cartridge or frame construction.

It may be conceivable that said cartridge or frame construction may function as an interface between the initially-mentioned functions of the device and the inflow and outflow liquids.

It may be conceivable that the cartridge or frame construction may be mountable and/or fixable to the rest of the system by mechanical means, e.g., by clamping means and/or locking means and/or latching means. However, it may additionally or alternatively be conceivable that the cartridge or frame construction may be mountable and/or fixable to the rest of the system by vacuum means, e.g., by vacuum means sucking the cartridge or frame construction against at least a part of the rest of the system thereby reliably mounting and/or fixing the cartridge or frame construction on and/or in the system.

It may be conceivable that the integrated liquid interface device may be formed as a hub device for connecting to the integrated inflow and/or outflow drain device, e.g., having a multi-lumen formed by at least the first tube arrangement and the second tube. The hub device may be configured for at least connecting to and/or combining one or more further effusion liquid inlets, e.g., a/the second effusion liquid inlet, a/the third effusion liquid inlet and/or a/the fourth effusion liquid inlet. It may be conceivable that the hub device may comprise at least one fluid connector piece, e.g., a Y-shaped connector, for combining effusion liquid streams receivable via the corresponding effusion liquid inlets. The hub device may comprise at least one injection port for injecting additional material in the infusion liquid, such as clot dissolvent, e.g., in case of excessive clotting further downstream in the system.

It may be conceivable that the hub device may be disposable, e.g., configured as a disposable hub device.

It may be conceivable that the system comprises a hub receiving device for holding the hub device. The hub receiving device may be formed as a cradle device. The hub receiving device may include and/or house the temperature sensor of the system, e.g., to measure a/the temperature of infusion liquid, e.g., for controlling the inflow by the control unit.

It may be conceivable that the hub receiving device may be non-disposable, i.e., reusable, e.g., may be configured as a non-disposable hub receiving device.

It may be conceivable that the hub receiving device may include and/or house the heater device of the system, e.g., to heat the infusion liquid being intended to be guided to the wound and/or the body cavity, in particular the pericardial and/or pleural cavity, to a desired infusion liquid temperature.

It may be conceivable that the system comprises a slope device, wherein the slope device may be formed by the tubes of the system connecting the system to the wound and/or the body cavity, in particular the pericardial and/or pleural cavity, wherein the slope device may be formed such that said tubes are sloped from the wound and/or the body cavity, in particular the pericardial and/or pleural cavity, towards the system.

In other words, the slope device may facilitate a height difference between a patient and at least a part of the system, e.g., most of the system, (with respect to the gravity), wherein the patient may generally be arranged on a higher position.

In particular the tubes forming the slope device may be arranged such as to be sloped in a linear or circular or spiral manner, e.g., in a substantially downward oriented and meander manner and/or in a substantially downward oriented and spiral manner and/or in a substantially downward oriented and screw manner. Accordingly, a good flow in the tubing may thereby be facilitated, in particular, without sags where the fluid may possibly accumulate in the tubing.

It should be understood that connecting elements and connecting lines (e.g., connecting tubes) between the above-described elements for guiding infusion and/or effusion liquid may also be included in the flushing system and may accordingly be part of the flushing system.

The invention will be better understood with the aid of the description of an embodiment given by way of example and illustrated by the figures.

It is schematically shown in
- **Fig. 1**: a flushing system according to an embodiment of the present invention;
- **Figs. 2A and 2B**: an integrated inflow and/or outflow drain device for a flushing system according to an embodiment of the present invention;
- **Figs. 3A and 3B**: an integrated inflow and/or outflow drain device for a flushing system according to an embodiment of the present invention;
- **Fig. 4**: an integrated inflow and/or outflow drain device for a flushing system according to an embodiment of the present invention;
- **Fig. 5**: an integrated inflow and/or outflow drain device for a flushing system according to an embodiment of the present invention;
- **Fig. 6**: a flushing system according to another embodiment of the present invention;
- **Fig. 7**: a flushing system according to another embodiment of the present invention;
- **Fig. 8**: a table showing information and/or notifications providable to a user by the flushing system of Fig. 1, 6, 7 and/or 9;
- **Fig. 9**: a flushing system according to another embodiment of the present invention;
- **Fig. 10**: a component of a flushing system according to another embodiment of the present invention;
- **Fig. 11**: a top view of the component of **Fig. 10****;**
- **Fig. 12**: an integrated inflow and/or outflow drain device for a flushing system according to an embodiment of the present invention;
- **Fig. 13**: a cross-sectional view of the integrated inflow and/or outflow drain device of **Fig. 12****;**
- **Fig. 14**: an integrated inflow and/or outflow drain device for a flushing system according to an embodiment of the present invention;
- **Fig. 15**: a portion of an integrated inflow and/or outflow drain device for a flushing system according to an embodiment of the present invention;
- **Fig. 16**: a cross-section of an integrated inflow and/or outflow drain device for a flushing system according to an embodiment of the present invention; and
- **Fig. 17**: a cross-section of an integrated inflow and/or outflow drain device for a flushing system according to an embodiment of the present invention.

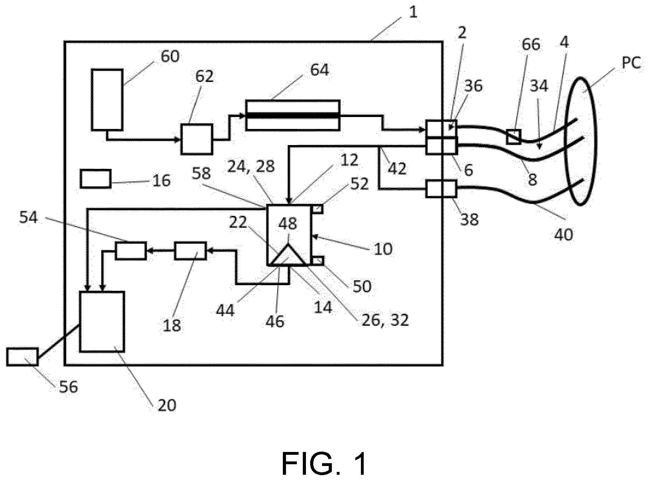

Turning to **Fig. 1****,** a flushing system 1 configured to flush a body cavity, in particular here the pericardial cavity PC, of a patient is schematically shown.

The system 1 comprises an infusion liquid outlet 2.

The infusion liquid outlet 2 is configured to connect to a first tube arrangement 4.

The first tube arrangement 4 has a plurality of (e.g., at least two, e.g., two or more) infusion liquid lumen 100 to guide a flow of infusion liquid from the system 1 to the pericardial cavity PC.

Each infusion liquid lumen 100 of the first tube arrangement 4 has at least one infusion liquid opening 102 (not shown in **Fig. 1**) for leading the infusion liquid into the pericardial cavity PC.

The infusion liquid openings 102 are arranged along the first tube arrangement 4 and are spaced from one another (not shown in **Fig. 1**).

The system 1 comprises an effusion liquid inlet 6.

The effusion liquid inlet 6 is configured to connect to a second tube 8.

The second tube 8 has an effusion liquid lumen 104 to guide the effusion liquid flow from the pericardial cavity PC to the system 1.

The system 1 comprises a buffer container 10.

The buffer container 10 has a buffer volume.

The buffer container 10 comprises a buffer inlet 12 and a first buffer outlet 14.

The buffer inlet 12 is configured to receive effusion liquid from the pericardial cavity PC.

The first buffer outlet 14 is configured to output the received effusion liquid.

The system 1 comprises a flow rate control system.

The flow rate control system is configured for controlling the flow rate (e.g., a volume) of the effusion liquid output from the buffer container 10.

The flow rate control system comprises a control unit 16 and a first pump device 18.

The control unit 16 is configured to provide one or more control signals.

The first pump device 18 is configured for pumping effusion liquid from the buffer container 10 to (e.g., towards) one or more effusion liquid container 20 at an effusion liquid flow rate. The effusion liquid flow rate (e.g., the volume) is adjustable by the control signals of the control unit 16.

In the present embodiment, the first pump device 18 is a peristaltic pump. However, it is also conceivable that other kinds of pumping devices are useable. For example, it may additionally or alternatively be conceivable, that the first pump device 18 may comprise a volumetric pump, a membrane pump, an impeller pump and/or a syringe pump device including a syringe pump and, optionally, at least two valves.

The buffer container 10 comprises a clot trap 22.

The clot trap 22 is configured for preventing clots included in the effusion liquid being received from the pericardial cavity PC from exiting the buffer container 10 via the first buffer outlet 14.

The buffer container 10 has a cylindrical shape extending along a central axis (not shown in the figures), which substantially extends along the gravitational direction. However, any other shape may be conceivable.

The buffer container comprises a top end 24 and a bottom end 26.

Further, the buffer container 10 comprises a top wall 28, a side wall 30 and a bottom wall 32.

The buffer inlet 12 is disposed closer to the top wall 28 than to the bottom wall 32, e.g., with respect to the gravitational direction.

The first buffer outlet 14 is disposed closer to the bottom wall 32 than to the top wall 28.

In the present embodiment, the buffer inlet 12 is arranged in the top wall 28 of the buffer container 10 and the first buffer outlet 14 is arranged in the bottom wall 32.

The flushing system 1 is configured to provide approximately 50 to 1000 ml of infusion liquid per hour, in particular approximately 500 ml of infusion liquid per hour. Hence, the buffer volume of the buffer container 10 is configured (e.g., sized) to be able to handle approximately 500 ml of effusion liquid per hour as well as sudden gushes of 400 ml or 500 ml of effusion liquid per 5 min.

The system 1 comprises a flow rate control system.

The flow rate control system is configured for controlling the flow rate (e.g., a volume) of the infusion liquid towards the pericardial cavity PC, i.e., towards the infusion liquid outlet 2.

The flow rate control system comprises a control unit 16 and a second pump device 62.

The control unit 16 is configured to provide one or more control signals.

The pump device 62 is configured for pumping infusion liquid towards the pericardial cavity PC, i.e., towards the infusion liquid outlet 2.

The effusion liquid flow rate (e.g., the volume) is adjustable by the control signals of the control unit 16. In other words, the control unit 16 can control the flow rate of infusion liquid that is pumped to the pericardial cavity PC.

In the present embodiment, the second pump device 62 is a peristaltic pump. However, it is also conceivable that other kinds of pumping devices are useable. In particular, the second pump device 62 may be any suitable pump device for infusion liquid, that preferably allows pumping at a relative accurate flow rate.

The flushing system 1 is configured to provide approximately 500 ml of infusion liquid per hour. Hence, the second pump device 62 is configured (e.g., sized) to pump approximately 500 ml of infusion liquid per hour. Further, the flushing system 1 is also configured to be able to handle sudden gushes of 400 ml of effusion liquid per 5 min. As can be seen in **Fig. 1**, the first tube arrangement 4 and the second tube 8 are combined in an integrated inflow and/or outflow drain device 34.

The first tube arrangement 4 is integrated in the second tube 8 in such a way that the first tube arrangement 4 does not consume lumen of the second tube 8. In the present case, the first tube arrangement 4 is positioned in the wall of the second tube 8.

Further details concerning the integrated inflow and/or outflow drain device 34, the first tube arrangement 4 and the second tube 8 are given further below.

As can be further seen in **Fig. 1**, the infusion liquid outlet 2 and the effusion liquid inlet 6 are combined in an integrated liquid interface device 36, which is configured to connect to the integrated inflow and/or outflow drain device 34.

In other words, the integrated liquid interface device 36 is configured as a corresponding counterpart to the integrated inflow and/or outflow drain device 34 for connecting with each other.

Additionally, in the present embodiment, the system 1 comprises a further effusion liquid inlet 38.

The further effusion liquid inlet 38 is configured to connect to a further tube 40.

The further tube 40 has an effusion liquid lumen to guide the effusion liquid flow from the pericardial cavity PC to the system 1.

In this embodiment, the effusion liquid inlet 6 can be referred to as a/the first effusion liquid inlet 6 and the further effusion liquid inlet 38 can be referred to as a/the second effusion liquid inlet 38, and the further tube 40 can be referred to as a/the third tube 40.

The effusion liquid flows of the second and the third tubes 8, 40 can be merged by means of a fluid connector piece 42. The fluid connector piece 42 may be formed as a Y-shaped connector piece.

The effusion liquid inlet 6, i.e., the integrated liquid interface device 36, is connected to the fluid connector piece 42 (e.g., via a connecting line, for example, a connecting tube).

The second effusion liquid inlet 38 is connected to the fluid connector piece 42 (e.g., via a connecting line, for example, a connecting tube).

The fluid connector piece 42 is connected to the buffer inlet 12 (e.g., via a connecting line, for example, a connecting tube).

The first buffer outlet 14 is connected to the first pump device 18 (e.g., via a connecting line, for example, a connecting tube).

In the present embodiment, the clot trap 22 is a mechanical means. In particular, the clot trap 22 comprises a sieve structure 44. The sieve structure 44 is disposed between the buffer inlet 12 and the first buffer outlet 14 of the buffer container 10. The clot trap 22 may be integrally formed with the buffer container (e.g., on the bottom wall 32 of the buffer container 10). The sieve structure 44 comprises a plurality of openings formed therein. The openings are configured (e.g., sized) to stop clots from passing through the sieve structure 44.

The clot trap 22 has a conical shape. The conical shape has a base 46 facing towards the first buffer outlet 14 and an apex 48 facing the buffer inlet 12. Accordingly, the sieve structure 44 is at least partly or completely conically shaped. It is also possible, that the clot trap may have other shapes, e.g., a truncated cone shape, a dome shape, a flat shape etc.

The buffer container 10 further comprises at least one level sensor 50, 52.

The at least one level sensor 50, 52 is configured for obtaining at least one filling level of the buffer volume.

In the present embodiment, the buffer container 10 comprises a first level sensor 50 and a second level sensor 52 for obtaining a first filling level and a second filling level of the buffer volume, respectively.

The second level sensor 52 is arranged at a higher level than the first level sensor 50 with respect to the gravitational direction.

In the present embodiment, the second level sensor 52 is arranged adjacent to the top wall 28 and the first level sensor 50 is arranged adjacent to the bottom wall 32.

However, the buffer container 10 may comprise more than said two level sensors 50, 52 in order to obtain intermediate filling levels.

Additionally or alternatively, the first level sensor 50 and/or the second level sensor 52 may also be arranged at intermediate positions between the top and the bottom wall 28, 32.

Each of the level sensors 50, 52 are configured to obtain their respective filling level by optical means, as generally known in the art. However, any other suitable sensor may also be applicable (such as mechanical sensors, capacitive sensors, resistive sensors, ultrasonic sensors etc.).

The system 1 comprises a hematocrit sensor 54.

The hematocrit sensor 54 is configured to receive effusion liquid from the pericardial cavity PC, to measure a hematocrit value, and to pass the effusion liquid on (e.g., for further processing and/or further assignment).

In particular, the hematocrit sensor 54 is configured to measure the hematocrit value of the effusion liquid for obtaining a blood loss volume and/or a blood loss flow rate from the pericardial cavity PC.

Further, in particular, the hematocrit sensor 54 is configured to optically measure said hematocrit value.

In particular, the hematocrit sensor 54 is configured to optically measure the hematocrit value of the effusion liquid, which is received from the buffer container 10, for obtaining a blood loss volume and/or a blood loss flow rate from the pericardial cavity PC.

As can be seen in **Fig. 1**, the hematocrit sensor 54 is arranged at a connecting line (e.g., connecting tube) downstream from the first pump device 18.

The hematocrit sensor 54 is connected or connectable to (the) one or more effusion liquid containers 20 (e.g., via a connecting line, for example, a connecting tube).

Hence, the system further comprises said one or more effusion liquid containers 20, wherein, in the present embodiment, as an example, the system 1 comprises (only) one effusion liquid container 20.

In other words, further downstream with respect to the hematocrit sensor 54, the one or more effusion liquid containers 20, i.e., the (one) effusion liquid container 20, are (is) arranged.

In this respect, the hematocrit sensor 54 is arranged downstream from the buffer container 10 and upstream from the effusion liquid container 20.

In other words, as can be seen in **Fig. 1**, the hematocrit sensor 54 is arranged at a connecting line (e.g., connecting tube) downstream from the buffer container 10. Hence, the hematocrit sensor 54 is arranged downstream from the (first) effusion liquid inlet 6, i.e., the integrated liquid interface device 34, and downstream from the second effusion liquid inlet 38.

Said effusion liquid container 20 is connectable or, in the present embodiment, connected to an intensive care unit (ICU) vacuum wall connector 56.

The ICU vacuum wall connector 56 can be considered as a suction device for drawing effusion liquid out of the pericardial cavity PC by means of a relative low pressure.

Said suction device, i.e., the ICU vacuum wall connector 56, can be part of the system 1 or can be assigned to the system 1 (i.e., may not explicitly be a part of the system).

The effusion liquid container 20 is accordingly connectable or, in the present embodiment, connected to the buffer container 10 via the hematocrit sensor 54, the first pump device 18 and their corresponding connecting lines, which connect each of said elements 10, 18, 20, 54. Hence, an indirect connection is provided.

Additionally, the effusion liquid container 20 has a direct connection with the buffer container 10, which is described below.

Referring again to the buffer container 10, the buffer container 10 comprises a second buffer outlet 58.

The second buffer outlet 58 is configured to output effusion liquid (e.g., surplus effusion liquid that can not be processed timely (either the gush is too large or fast, or the system is blocked somewhere)) and/or air.

The second buffer outlet 58 is connectable or, in the present embodiment, connected to the effusion liquid container 20 (e.g., by a bypass line, for example, a bypass tube).

Hence, said direct connection may be provided between the effusion liquid container 20 and the buffer container 10, in particular, to thereby provide safety in case of any occlusion of the system by clots in the buffer container and/or occlusion of said pump and/or any lines.

The second buffer outlet 58 is arranged at a higher level than the first buffer outlet 14 and at a lower level than the buffer inlet 12, each with respect to the gravitational direction.

The second buffer outlet 58 is disposed on an opposite side of the clot trap 22 with respect to the first buffer outlet 14. Hence, the second buffer outlet 58 is disposed on the same side as the buffer inlet 12.

The second buffer outlet 58 may act as a safety overflow from the buffer container 10, to thereby provide an open connection to the effusion liquid container 20 for safety reasons (e.g., for preventing back pressure /afflux). In particular, the open connection may be understood as an always-open connection.

The second buffer outlet 58 may provide an exit path for air being separated from the effusion liquid in the buffer container 10.

Now referring to the remaining elements of **Fig. 1** particularly disposed upstream with respect to the pericardial cavity PC.

In this regard, the system 1 comprises one or more infusion liquid containers 60.

The one or more infusion liquid containers 60 are configured for containing infusion liquid.

For example, the one or more infusion liquid containers 60 may be a number of bags containing saline fluid, such as NaCI 0,9%, as known in the art.

In the present embodiment, as an example, the system 1 comprises one infusion liquid container 60.

The infusion liquid container 60 is connectable or, in the present embodiment, connected to the second pump device 62 (e.g., via a connecting line, for example, a connecting tube).

The second pump device 62 is configured for pumping infusion liquid from the infusion liquid container 60 towards the infusion liquid outlet 2, i.e., in the present embodiment, towards the integrated liquid interface device 36, at an infusion liquid flow rate. The infusion liquid flow rate is adjustable by the control signals of the control unit 16.

The system 1 further comprises a heater device 64.

The heater device 64 is disposed downstream from the second pump device 62 and connected thereto (e.g., via a connecting line, for example, a connecting tube).

The heater device 64 is configured to heat the infusion liquid passing therethrough.

The heater device 64 may be part of a temperature control system of the system 1 (not shown in the figures). The temperature control system may be configured to control a temperature of the infusion liquid flow. Further, the temperature control system may comprise a temperature sensor for obtaining a body temperature and/or a pericardial cavity temperature.

The heater device 64 is connected to the infusion liquid outlet 2, i.e., in the present embodiment, to the integrated liquid interface device 36 (e.g., by a connecting line), thereby allowing to provide the temperature controlled infusion liquid to the pericardial cavity PC.

A pressure sensor 66 is also included in the system 1 of **Fig. 1****.**

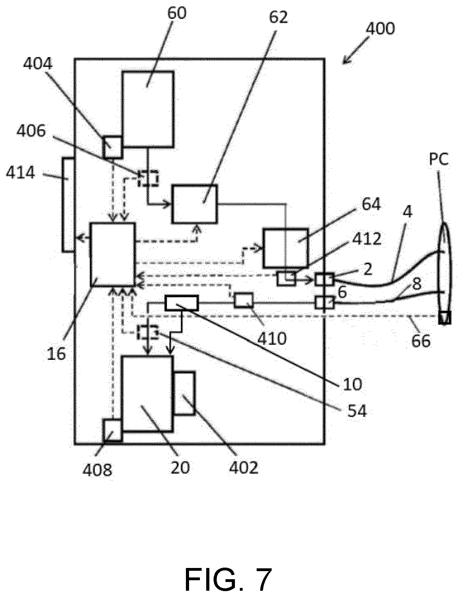

The pressure sensor 66 is provided in the first tube arrangement 4. However, the pressure sensor 66 may also be positioned in connection with the first tube arrangement 4 or inside or in connection with the second tube 8 or the pericardial cavity PC (e.g., as shown in **Fig. 7**).

The pressure sensor 66 is configured for obtaining the pressure in the pericardial cavity PC. In other words, the pressure sensor 66 is configured to provide a pressure signal representative for the pressure in the pericardial cavity PC.

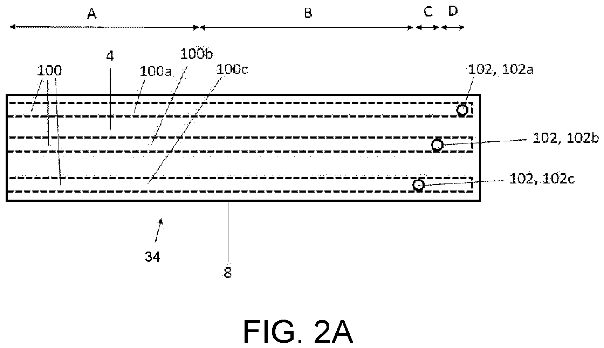

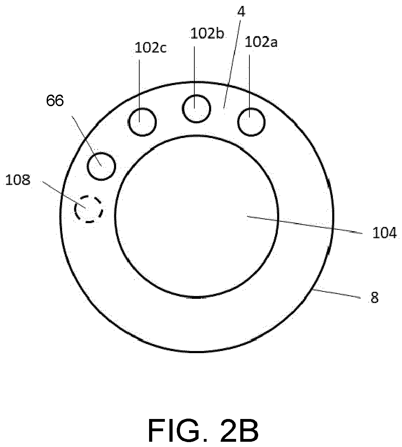

With reference to **Figs. 2A** **and** **2B****,** an integrated inflow and/or outflow drain device 34 for a flushing system 1 according to an embodiment of the present invention is now described in more detail. For example, the integrated inflow and/or outflow drain device 34 shown in **Figs. 2A** **and** **2B** may be the integrated inflow and/or outflow drain device 34 of **Fig. 1****.**

As can be seen in **Figs. 2A** **and** **2B****,** the integrated inflow and/or outflow drain device 34 comprises the first tube arrangement 4 and the second tube 8.

The first tube arrangement 4 has a plurality of infusion liquid lumen 100. In the present exemplary embodiment, the first tube arrangement 4 has three infusion liquid lumens 100 being a first infusion liquid lumen 100a, a second infusion liquid lumen 100b and a third infusion liquid lumen 100c.

Each infusion liquid lumen 100 of the first tube arrangement 4 has at least one infusion liquid opening 102 for leading the infusion liquid into the pericardial cavity PC. In the present exemplary embodiment, each of the infusion liquid lumens 100a, 100b, 100c has one respective infusion liquid opening 102a, 102b, 102c. Hence, the first infusion liquid lumen 100a has a first infusion liquid opening 102a, the second infusion liquid lumen 100b has a second infusion liquid opening 102b, and the third infusion liquid lumen 100c has a third infusion liquid opening 102c.

The second tube 8 has an, e.g., one, effusion liquid lumen 104 for guide the effusion liquid flow from the pericardial cavity PC to the system 1.

The effusion liquid lumen 104 of the second tube 8 has at least one effusion liquid opening 106 (not shown in **Figs. 2A** **and** **2B****)** for receiving the effusion liquid from the pericardial cavity PC.

The first tube arrangement 4 and, accordingly, the first infusion liquid lumen 100a, the second infusion liquid lumen 100b and the third infusion liquid lumen 100c are integrated in the second tube 8 in such a way that the first tube arrangement 4 and, accordingly, the lumens 100a, 100b, 100c do not consume lumen of the second tube 8. In this respect, as can be seen in **Fig. 2B****,** the first tube arrangement 4 and, accordingly, the first infusion liquid lumen 100a, the second infusion liquid lumen 100b and the third infusion liquid lumen 100c are positioned in the wall of the second tube 8.

The first infusion liquid lumen 100a, the second infusion liquid lumen 100b and the third infusion liquid lumen 100c extend along the second tube 8 in a substantially parallel manner.

The infusion liquid openings 102a, 102b, 102c are arranged along the first tube arrangement 4 and, accordingly, the second tube 8.

The infusion liquid openings 102a, 102b, 102c are spaced from one another. In the present case, the first infusion liquid opening 102a is spaced from the second infusion liquid opening 102b by distance D, and the second infusion liquid opening 102b is spaced from the third infusion liquid opening 102c by distance C.

The distances C, D between the infusion liquid openings 102a, 102b, 102c are dimensioned such as to allow a predetermined distribution of infusion liquid inflows for flushing the pericardial cavity PC.

The effusion liquid opening(s) 106 are arranged along the second tube 8 and may be spaced from one another.

The effusion liquid opening(s) 106 are spaced from the infusion liquid openings 102a, 102b, 102c by at least distance B.

The distance B is dimensioned such as to allow that the effusion liquid openings 106 and the infusion liquid openings 102a, 102b, 102c are arrangeable at different levels with respect to the gravitational direction. For example, the distance B may be dimensioned such as to allow that the effusion liquid openings 106 and the infusion liquid openings 102a, 102b, 102c are arranged or arrangeable at different levels with respect to the gravitational direction, when the integrated inflow and/or outflow drain device 34 is bend, e.g., into a substantially U-formed shape.

Further, the effusion liquid opening(s) 106 are spaced from the effusion liquid inlet 6 and, accordingly, the integrated liquid interface device 36 by at least distance A.

The distance A is dimensioned such as to bridge at least a distance between the pericardial cavity PC and the integrated liquid interface device 36. In other words, the distance A is dimensioned such as to bridge at least a distance between a patient and the flushing system 1.

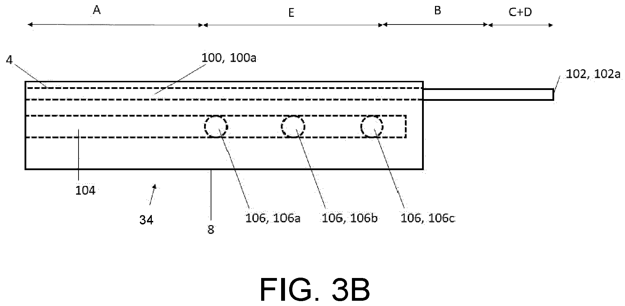

The effusion liquid openings 106 are larger (e.g., with respect to a footprint area and/or a diameter) than the infusion liquid openings 102a, 102b, 102c, respectively (not shown in **Figs. 2A** **and** **2B**; confer, e.g., **Fig. 3B**).

In this respect, the different lengths serve the purpose of positioning the effusion liquid openings 106 and the infusion liquid openings 102a, 102b, 102c at such distance from each other, i.e., distance B, in order to prevent the inflow liquid from leaving the pericardial cavity PC immediately by the shortest pathway out of the pericardial cavity PC through the outflow tube 8 and thereby not flushing the pericardial cavity.

As can be seen in **Fig. 2A**, the infusion liquid openings 102a, 102b, 102c are substantially positioned at a proximal end and/or portion of the integrated inflow and/or outflow drain device 34 and the effusion liquid opening(s) 106 are substantially positioned at an intermediate portion of the integrated inflow and/or outflow drain device 34. However, it should be understood that the infusion liquid openings 102a, 102b, 102c may substantially be positioned at an intermediate portion of the integrated inflow and/or outflow drain device 34 and the effusion liquid opening(s) 106 may substantially be positioned at a proximal end and/or portion of the integrated inflow and/or outflow drain device 34.

As already outlined above with respect to **Fig. 1**, the system 1 further comprises a/the pressure sensor 66 being positioned with the first tube arrangement 4.

As can be seen in **Fig. 2B****,** the pressure sensor 66 is integrated in the second tube 8 together with the first tube arrangement 4. In this respect, the pressure sensor 66 is arranged in such a way that no lumen is consumed in the second tube 8.

In particular, the pressure sensor 66 is positioned in the wall of the second tube 8, e.g., next to the first tube arrangement 4.

Accordingly, since the pressure sensor 66 is integrated in the integrated inflow and/or outflow drain device 34, measuring of an exact pressure in the pericardial cavity PC can be performed, e.g., at the proximal end of the integrated inflow and/or outflow drain device 34 and/or at the first tube arrangement 4.

Optionally, the integrated inflow and/or outflow drain device 16 may comprise a temperature sensor 108, e.g., the temperature sensor of the optional temperature control system described in connection with **Fig. 1****.**

In this respect, the temperature sensor 108 may be integrated in the integrated inflow and/or outflow drain device 34 in order to provide an exact measurement of a central body temperature.

Turning back to **Fig. 1**, generally, the flushing system 1 is configured for continuous flushing of the pericardial cavity PC, in particularly as a post-operative treatment. During continuous flushing of the pericardial cavity PC, infusion liquid is pumped into the cavity PC to dilute blood and clots present in the cavity PC. At the same time effusion liquid, i.e. a mixture of blood and infusion liquid mixed in the pericardial cavity PC, is drawn out of the cavity PC in order to remove the blood and clots from the cavity PC.

The liquid drawn out of the pericardial cavity PC, i.e., the effusion liquid can be guided in the direction of the hematocrit sensor 54.

In particular, the liquid drawn out of the pericardial cavity PC, i.e., the effusion liquid can be guided in the direction of the hematocrit sensor 54 via the buffer container 10.

In particular, the effusion liquid can be guided from the pericardial cavity PC via the integrated inflow and/or outflow drain device 34.

Since the first tube arrangement 4 is integrated in the second tube 8 in such a way that the first tube arrangement 4 does not consume lumen of the second tube 8 and, in particular, since the first tube arrangement 4 is positioned in the wall of the second tube 8 in the present exemplary case, the integrated inflow and/or outflow drain device 34 may be allowed to have a regular shape, such as a rounded or circular shape, to thereby be placeable or be placed in the patient's body without any leakage from the pericardial cavity PC. In other words, the integrated inflow and/or outflow drain device 34 may have a regular shape, such as a rounded or circular shape.

The effusion liquid flows of the second and the third tubes 8, 40 can be merged by means of the before-mentioned fluid connector piece 42, in order to allow the effusion liquid to be subsequently guided to the buffer container 10 and then to the hematocrit sensor 54 (e.g., by the connecting line, which connects the buffer container 10 with the hematocrit sensor 54).

By means of the hematocrit sensor 54, the effusion liquid is analyzed (i.e., measured) with respect to its hematocrit value while being guided further towards the one or more effusion liquid containers 20, i.e., in the present embodiment, to the effusion liquid container 20.

Hence, measuring the hematocrit value of the effusion liquid by the hematocrit sensor 54 for obtaining a blood loss volume and/or a blood loss flow rate from the pericardial cavity PC can be accurately performed.

After passing the hematocrit sensor 54, the effusion liquid is guided to the effusion liquid container 20.

When the system 1 is in operation, before the infusion liquid may enter the patient's body or the patient's pericardial cavity PC, the infusion liquid is preferably temperature controlled to a desired infusion liquid temperature.

Accordingly, the system 1 comprises the above-described heater device 64, which, in the present embodiment, is disposed downstream from the second pump device 62.

The heater device 64 is configured to heat the infusion liquid, which is to be guided to the pericardial cavity PC, to a desired infusion liquid temperature.

The desired infusion liquid temperature is based on a temperature of the pericardial cavity PC. Preferably, the infusion liquid temperature is in a range of 36°C and 38°C, more preferably approximately 37°C. However, the desired infusion liquid temperature may also be adapted or adjusted to the actual body temperature of the patient, and/or the desired infusion liquid temperature may be adapted to rewarm and/or cool down the patient, e.g., as required by a/the clinical situation.

Accordingly, the heater device 64 allows to provide the temperature controlled infusion liquid to the pericardial cavity PC.

Further, as described above, the pressure sensor 66 can obtain the pressure in the pericardial cavity PC. In other words, the pressure sensor 66 can provide a pressure signal representative for the pressure in the pericardial cavity PC.

The obtained pressure can be provided to the control unit 16, wherein the control unit 16 is configured to adjust the infusion liquid flow rate (by controlling the second pump device 62) to keep the pressure (i.e., for example, the pressure signal) within desired pressure limits so as to avoid acute cardiac tamponade.

In a (post)operative procedure, a patient is usually provided with two pericardial cavity drains for draining postoperative blood loss, which, with respect to the present embodiment, can be the second tube 8 and the third tube 40. The second tube 8 can be a part of the above-described integrated inflow and/or outflow drain device 34.

The infusion liquid, which is warmed to the desired infusion liquid temperature, e.g., to body temperature, by the heater device 64, is automatically pumped to the pericardial cavity PC through the first tube arrangement 4, which is also a part of the above-described integrated inflow and/or outflow drain device 34, by means of the second pump device 62. Thereby, the pericardial cavity PC is continuously flushed.

The first tube arrangement 4 is arranged in a substantially horizontal orientation within the pericardial cavity PC in order to lead the infusion liquid therein. Accordingly, the infusion liquid may be uniformly and smoothly guided into the pericardial cavity PC. Hence, this rainforest-shower-like delivery of infusion liquid can thereby provide an enhanced flushing performance.

In addition, the first tube arrangement 4 and the second tube 8 are arranged such that the effusion liquid openings 106 and the infusion liquid openings 102a, 102b, 102c are disposed at different levels with respect to the gravitational direction for allowing the infusion liquid to either work with or against gravity, when applied in the pericardial cavity PC. Hence, the gravitational force may either support the flushing of the pericardial cavity, in case of providing the liquid at a higher level than draining the liquid, or may allow for a more uniform and smoother flushing of the pericardial cavity, in case of providing the liquid at a lower level than draining the liquid. In the present case, with regards to **Figs. 1 to 2B**, the first tube arrangement 4 and the second tube 8 are arranged such that the effusion liquid openings 106 and the infusion liquid openings 102a, 102b, 102c are disposed at different levels with respect to the gravitational direction for allowing the infusion liquid to work with the gravity, when applied in the pericardial cavity PC. In particular, the first tube arrangement 4 is arranged at a higher level than the second tube 8 with respect to the gravitational direction.

Generally, the standard flushing volume is 500 ml per hour and can be adjusted if necessary. Medical staff is continuously informed on patient status through a graphical user interface (which may also be included in the system 1, e.g., in form of a display unit), in particular on a trend and/or absolute measure of blood loss (volume / time unit).

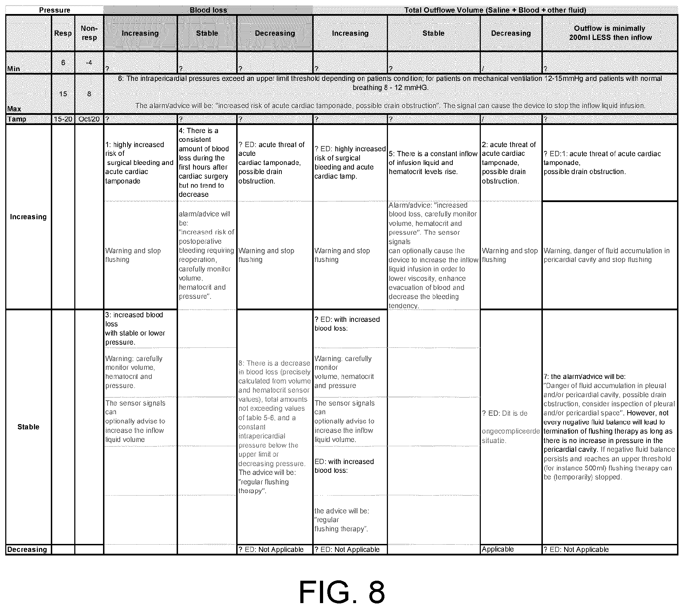

In particular, in this respect, there may be notifications and/or information providable to the medical staff as shown in the table of **Fig. 8****.** Preferably, there may be notifications and/or information if the trend of column exceeds the values as indicated in the table of **Fig. 8****.**

The drained fluids, i.e., the effusion liquid, are drained via the second and the third tubes 8, 40 towards the hematocrit sensor 54.

Due to the buffer container 10, the effusion liquid is buffered before being guided further for subsequent proceedings. Further, due to the clot trap 22, the effusion liquid can be freed from clots and/or air, which may be included in the effusion liquid.

In other words, clots can be separated from the effusion liquid by means of the clot trap 22.

In particular, since the clot trap 22 comprises the sieve structure 44 being disposed between the buffer inlet 12 and the first buffer outlet 14 of the buffer container 10, clots are prevented from passing through the first buffer outlet 14.

When the system 1 is operated, each of the level sensors 50, 52 can obtain their respective filling level of the buffer volume.

The control unit 16 provides at least one of the (e.g., its) control signals on the basis of the obtained filling level of the buffer volume, to thereby control the first pump device 18 and adjust the effusion liquid rate from the buffer container. Further, this also allows to provide single liquid batches to the subsequent hematocrit sensor 54 (i.e., the hematocrit sensor, which is disposed downstream from the first pump device 18) for analysis.

In this respect, the control unit 16 is configured to control the first pump device 18 in order to pump a predetermined, i.e., a known, volume on the basis of said control signals.

For example, the control unit 16 can control the first pump device 18 in order to pump a volume of, e.g., 30 ml, which may be referred to as said batch. However, any other volume may be pumped on the basis of the control signals of the control unit 16.

Hence, the buffer container 10 collects effusion liquid until the first pump device 18 is allowed to pump.

Then, a steady flow of effusion liquid can be generated in a controlled manner. As a consequence, subsequent analysis can be performed in/at the effusion liquid in a more accurate manner.

As already described above, the first pump device 18 is configured to perform a continuous and/or a discontinuous pumping action (e.g., for continuous analysis or discontinuous (i.e., batch) analysis of the effusion liquid) and is configured for gently conveying of sensitive material such as blood cells, which could be destroyed by rapidly moving or rotating elements, which might be part of other kinds of pumps.

Subsequently, the effusion liquid passes through the hematocrit sensor 54 towards the effusion liquid container 20 (supported by the low pressure provided by the ICU vacuum wall connector 56).

The effusion liquid is measured with respect to its hematocrit value by the hematocrit sensor 54 while passing therethrough.

The measured hematocrit value can be an important basis for deriving the blood loss.

When the pericardial pressure and blood loss values or their trends reach critical values, the system 1 can give a warning to the medical staff (e.g., via a signal provided by the control unit 16, for example, via a graphical user interface) in order to adjust the postoperative procedure.

In particular, in this respect, there may be notifications and/or information providable to the medical staff as shown in the table of **Fig. 8****.**

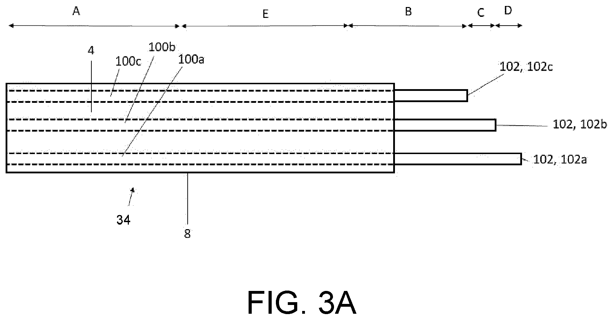

Now turning to **Figs. 3A** **and** **3B****,** another integrated inflow and/or outflow drain device 34 for a flushing system 1 according to an embodiment of the present invention is schematically shown. For example, the integrated inflow and/or outflow drain device 34 shown in **Figs. 3A** **and** **3B** may be the integrated inflow and/or outflow drain device 34 of **Fig. 1****.**

The integrated inflow and/or outflow drain device 34 of **Figs. 3A** **and** **3B** is substantially configured as the integrated inflow and/or outflow drain device 34 of **Fig. 2A** **and** **2B** described above. Hence, only the differences shall be described in the following.

The effusion liquid lumen 104 of the second tube 8 has a plurality of effusion liquid openings 106 for receiving the effusion liquid from the pericardial cavity PC, in particular, in the present case, a first, a second and a third effusion liquid opening 106a, 106b, 106c.

The plurality of effusion liquid openings 106 are distributed and spaced to each other over a distance E along the second tube 8.

The distance E is disposed between the distance A being substantially defined as described above and the distance B being substantially defined as described above.

As can be seen in **Figs. 3A** **and** **3B****,** the first tube arrangement 4 is longer than the second tube 8. In particular, the first tube arrangement 4 extends from a proximal end of the second tube 8.

In this respect, the first tube arrangement 4 is dimensioned with respect to the second tube 8 such as to allow that the effusion liquid openings 106 are spaced from the infusion liquid openings 102a, 102b, 102c by at least distance B. In particular, the third effusion opening 106c is spaced from the third infusion liquid opening 102c by the distance B.

The distance B is dimensioned such as to allow that the effusion liquid openings 106a, 106b, 106c and the infusion liquid openings 102a, 102b, 102c are arrangeable at different levels with respect to the gravitational direction.

As can be seen in **Fig. 3B****,** the effusion liquid openings 106a, 106b, 106c are arranged in a substantially perpendicular manner with respect to the infusion liquid openings 102a, 102b, 102c. However, it should be understood that any suitable arrangement may be applicable.

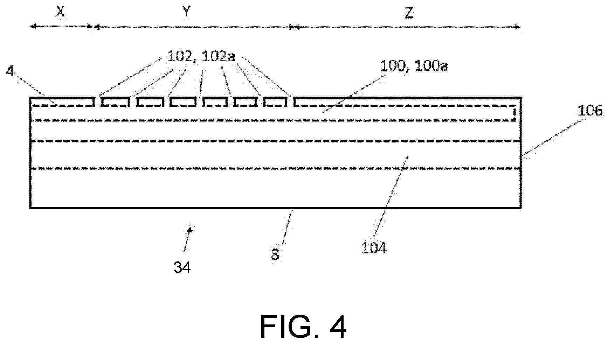

Turning to **Fig. 4****,** another integrated inflow and/or outflow drain device 34 for a flushing system 1 according to an embodiment of the present invention is schematically shown. For example, the integrated inflow and/or outflow drain device 34 shown in **Fig. 4** may be the integrated inflow and/or outflow drain device 16 of **Fig. 1****.**

The integrated inflow and/or outflow drain device 16 of **Fig. 4** is substantially configured as the respective integrated inflow and/or outflow drain devices 34 of **Fig. 2A** **and** **2B** and/or **Fig. 3A** **and** **3B** described above. Hence, only the differences shall be described in the following.

The first tube arrangement 4 has a plurality of infusion liquid lumens 100, 100a, wherein only one is depicted in **Fig. 4** for reasons of clarity.

Each infusion liquid lumen 100, 100a of the first tube arrangement 4 has a plurality of infusion liquid openings 102a for leading the infusion liquid into the pericardial cavity PC.

The plurality of infusion liquid openings 102a are distributed and spaced to each other over a distance Y along the first tube arrangement 4 and, accordingly, since the first tube arrangement 4 is integrated into the second tube 8, along the second tube 8.

The second tube 8 has an, e.g., one, effusion liquid lumen 104 for guide the effusion liquid flow from the pericardial cavity PC to the system 1.

The effusion liquid lumen 104 of the second tube 8 has at least one effusion liquid opening 106 for receiving the effusion liquid from the pericardial cavity PC.

The effusion liquid opening 106 is spaced from the infusion liquid openings 102a, 102b, 102c by at least distance Z.

The distance Z is dimensioned such as to allow that the effusion liquid opening 106 and the infusion liquid openings 102a are arrangeable at different levels with respect to the gravitational direction. For example, the distance Z may be dimensioned such as to allow that the effusion liquid opening 106 and the infusion liquid openings 102a are arranged or arrangeable at different levels with respect to the gravitational direction, when the integrated inflow and/or outflow drain device 34 is bend, e.g., into a substantially U-formed shape.

Further, the infusion liquid openings 102a (e.g., the group of infusion liquid openings 102a) are spaced from the infusion liquid outlet 2 and/or the effusion liquid inlet 6 and, accordingly, the integrated liquid interface device 36 by at least distance X.

The distance X is dimensioned such as to bridge at least a distance between the pericardial cavity PC and the integrated liquid interface device 36. In other words, the distance X is dimensioned such as to bridge at least a distance between a patient and the flushing system 1.

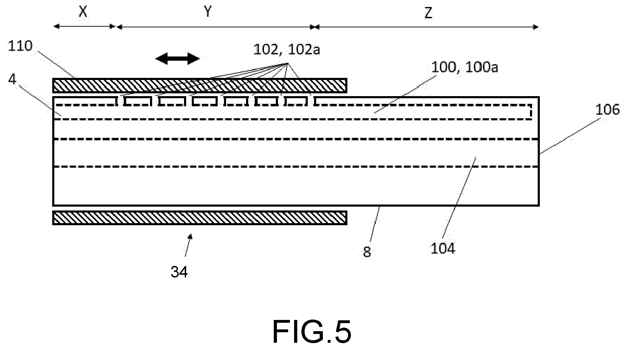

Turning to **Fig. 5****,** another integrated inflow and/or outflow drain device 34 for a flushing system 1 according to an embodiment of the present invention is schematically shown. For example, the integrated inflow and/or outflow drain device 34 shown in **Fig. 5** may be the integrated inflow and/or outflow drain device 34 of **Fig. 1****.**

The integrated inflow and/or outflow drain device 34 of **Fig. 5** is substantially configured as the respective integrated inflow and/or outflow drain devices 34 of **Figs. 2A to 4****,** in particular of **Fig. 4****,** described above. Hence, only the differences shall be described in the following.

The first tube arrangement 4 includes a retractable sheath 110. Accordingly, since the first tube arrangement 4 is integrated into the second tube 8, the retractable sheath 110 is also assigned to the second tube 8 and, hence, to the integrated inflow and/or outflow drain device 34.

The retractable sheath 110 is retractable between a first position and a second position via at least one intermediate position, along the first tube arrangement 4 and, hence, the second tube 8 and, accordingly, the integrated inflow and/or outflow drain device 34 (schematically shown by the arrow above the device 34).

In the first position, the infusion liquid openings 102a can be covered by the sheath 110 (exemplarily depicted in **Fig. 5**).

In the second position, the infusion liquid openings 102a are not covered by the sheath 110 (not shown in the figures).

In the intermediate position, some of the infusion liquid openings 102a can be covered by the sheath 110 (not shown in the figures).

In this respect, the sheath 110 is configured to extend along the first tube arrangement 4, the second tube 8 and/or the integrated inflow and/or outflow drain device 34.

When the flushing system 1 is operated, the sheath 110 may be used for adjusting the inflow of infusion liquid into the pericardial cavity PC by partly or completely covering the infusion liquid openings 102a. Accordingly, the flushing of the pericardial cavity PC may be performed in a more precise and/or effective manner.

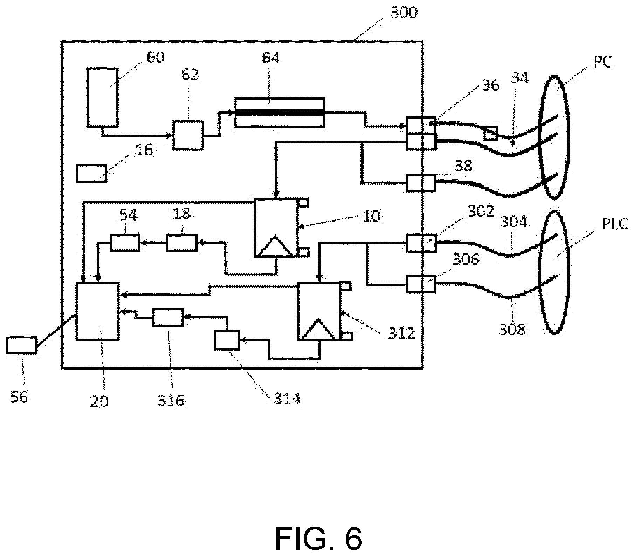

Turning to **Fig. 6****,** a flushing system 300 according to another embodiment is schematically shown. The flushing system 300 is substantially configured as the flushing system 1 of **Fig. 1** described above. Hence, only the differences shall be described in the following.

In this respect, the system 300 of **Fig. 6** comprises the integrated inflow and/or outflow drain device 34 as described with respect to **Fig. 1****.** However, it should be understood, that any of the devices described in connection with **Figs. 2A to 5** may be used in the system 300 of **Fig. 6****.**

The flushing system 300 is additionally configured for draining the pleural cavity PLC of the patient during flushing the pericardial cavity PC.

Hence, the system 300 further comprises a third effusion liquid inlet 302 and a fourth effusion liquid inlet 306.

The third effusion liquid inlet 302 is configured to connect to a fourth tube 304.

The fourth tube 304 has an effusion liquid lumen to guide the effusion liquid flow from the pleural cavity PLC to the system 300.

The fourth effusion liquid inlet 306 is configured to connect to a fifth tube 308.

The fifth tube 308 has an effusion liquid lumen to guide the effusion liquid flow from the pleural cavity PLC to the system 300.

The third effusion liquid inlet 302 and the fourth effusion liquid inlet 306 are connected to a fluid connector piece 310, such as a Y-shaped connector piece, (e.g., via a respective connecting line, for example, a respective connecting tube).

The effusion liquid flows of the fourth and the fifth tubes 304, 308 are mergeable or, when the system 300 is operated, merged by means of the fluid connector piece 310.

The system 300 comprises a further buffer container 312.

The further buffer container 312 is assigned to the liquid being received from the pleural cavity PLC.

Said further buffer container 312, which can be referred to as the second buffer container 312, is configured as the above-described buffer container 10.

The second buffer container 312 is connected to the fluid connector piece 310 (e.g., via a connecting line, for example, a connecting tube).

The system 300 comprises a further pump device 314, i.e., a third pump device 314, and a further hematocrit sensor 316, i.e., a second hematocrit sensor 316.

Each of said further elements 312, 314, 316 of the system 300 are configured as outlined above with respect to the buffer container 10, the first pump device 18 and the hematocrit sensor 54, respectively.

The second buffer container 312, the third pump device 314 and the second hematocrit sensor 316 are arranged in a parallel effusion liquid line assigned to the effusion liquid received from the pleural cavity PLC.

The second buffer container 312, the third pump device 314 and the second hematocrit sensor 316 are correspondingly arranged in the same manner as outlined above with respect to the buffer container 10, the first pump device 18 and the hematocrit sensor 54.

Accordingly, the second buffer container 312, the third pump device 314 and the second hematocrit sensor 316 are respectively connected via a respective connecting line, for example, a respective connecting tube.

In other words, the second hematocrit sensor 316 is arranged in a parallel effusion liquid line assigned to the effusion liquid received from the pleural cavity PLC. In still other words, the second hematocrit sensor 316 is configured to measure the hematocrit value of the effusion liquid for obtaining a blood loss volume and/or a blood loss flow rate (e.g., deriving) from the pleural cavity PLC.

Now turning to **Fig. 7****,** a flushing system 400 according to another embodiment is schematically shown. The flushing system 400 is substantially configured as the flushing system 1 of **Fig. 1** described above. Hence, only the differences shall be described in the following.

In this respect, the system 400 of **Fig. 7** comprises the integrated inflow and/or outflow drain device 34 as described with respect to **Fig. 1****.** However, it should be understood, that any of the devices described in connection with **Figs. 2A to 5** may be used in the system 400 of **Fig. 7****.**

When the system 400 is operated, the infusion liquid flow rate of the second pump device 62 is adjustable by the control unit 16 so that the control unit 16 can control the flow rate of infusion liquid that is pumped to the pericardial cavity PC based on signals from multiple sensors.

The effusion liquid received or receivable from the pericardial cavity PC is guided or guidable to the effusion liquid containers 20.

One or more suction devices 402 are arranged next to the effusion liquid container 20.

The suction devices 402 are configured to create a relative low pressure, i.e., an under pressure of for example -15 mmHg, in the effusion liquid containers 20 (which may also be performed by an ICU vacuum wall connector 56 as configured in the other embodiments).

This relative low pressure can be used to draw effusion liquid from the pericardial cavity PC towards the effusion liquid container 20.

A weight sensor 404 is provided to measure a change in weight of the infusion liquid in the infusion liquid container 60. This change in weight is representative for a decrease in the volume of infusion liquid in the infusion liquid container 60. On the basis of the change of volume of infusion liquid over the course of time, the flow rate of infusion liquid towards the pericardial cavity PC may be determined.

The weight sensor 404 or the control unit 16 may be configured to determine the flow rate of infusion liquid pumped out of the infusion liquid container 60. It is remarked that any other sensor configured to determine the flow rate of infusion liquid towards the pericardial cavity PC may be used. For example, the flow rate of infusion liquid may be determined by a flow rate sensor 406 which may be located anywhere in the infusion part of the system 400. It is remarked that the flow rate of infusion liquid may also be deducted from a control signal provided by the control unit 16 to adjust the flow rate of the second pump device 62 in response to one or more of the sensor signals herein. Any other way to determine the flow rate of infusion liquid may also be used.

Effusion liquid that flows out of the pericardial cavity PC due to the suction provided by the one or more suction devices 402 is received in the effusion liquid container 20 resulting in an increase of volume of effusion liquid in this effusion liquid container 20. This increase of volume in the effusion liquid container 20 may be determined with a weight sensor 408 configured to determine a volume/weight of effusion liquid in the effusion liquid container 20. On the basis of the change of volume/weight of effusion liquid over the course of time, the flow rate/volume of effusion liquid may be determined.

Any other sensor configured to determine the flow rate of effusion liquid from the pericardial cavity PC to the effusion liquid container 20 may also be used to calculate the flow rate/volume of effusion liquid. For example, the flow rate of effusion liquid may be determined by a flow rate sensor 410.

The sensors 404, 410, the control unit 16 and the second pump device 62 may form a part of the flow rate control system to control a flow rate of the infusion liquid flow at the infusion liquid outlet 2 on the basis of the sensor signals, the sensor signals comprising an infusion liquid signal representative for an infusion liquid flow rate towards the pericardial cavity PC and an effusion liquid signal representative for an effusion liquid flow rate from the pericardial cavity PC. The difference between the infusion liquid flow rate and the effusion liquid flow rate may generally correspond substantially to the blood loss flow rate of the patient from the pericardial cavity PC. Importantly, the control signals also comprise the hematocrit sensor signal from the hematocrit sensor 54 and the pressure signal from the pressure sensor 66.

On the basis of the calculated blood loss volume and/or flow rate, a suitable infusion liquid flow rate can be pumped by the second pump device 62.

Since it is important to accurately determine the actual blood loss volume from the pericardial cavity PC, the system 400 also comprises the above-described hematocrit sensor 54.

Additionally, the system 400 also comprises the above-described buffer container 10 (e.g., the above-described buffer container 10 of **Figs. 1** **and** **6**) being disposed upstream with respect to the hematocrit sensor 54 as shown in **Figs. 1** **and** **6****.**

The hematocrit sensor 54 generates a blood flow signal representative for the relative amount of blood or composition of the blood in the effusion liquid. The actual blood loss may be calculated from the outflow volume (e.g., outflow volumes; e.g., as described above and/or from the buffer container 10) and the hematocrit sensor values of the blood flow. This blood flow sensor signal is provided as an input to the control unit 16 in order to control the infusion flow rate of infusion liquid pumped towards the pericardial cavity PC.

In the present embodiment, the system 400 comprises a temperature control system comprising a temperature sensor 412 to measure a temperature of infusion liquid, the control unit 16 to provide a temperature control signal on the basis of the measured temperature of infusion liquid and a desired infusion liquid temperature, and the above-described heater device 64 that is controllable by the temperature control signal to heat the infusion liquid to the desired infusion liquid temperature. It is remarked that the control of the temperature control system does not have to be integrated in the control unit 16, but may also be formed by a separate control unit, for example a part of the heater device 64, or may be integrated in another control device or processing unit.

Since the control unit 16 can determine a blood loss volume or blood loss flow rate from the pericardial cavity PC of a patient based on input from the hematocrit sensor 54, this determined, in particular calculated, blood loss volume or blood loss flow rate may be used to monitor the blood loss from the pericardial cavity PC.

For example, the determined blood loss volume or blood loss flow rate may be displayed by a graphical user interface, e.g., a display device 414, so that a physician or nurse can easily monitor the development of the blood loss over time. Such display device 414 may also be used to display any other relevant parameter of the flushing process, such as used volume of infusion liquid, volume of received effusion liquid, flow rates of infusion liquid and effusion liquid, relative blood content in effusion liquid, composition of blood in effusion liquid, and trends thereof. Importantly, the display may show instructions based on the combined sensor input as explained elsewhere.

The blood loss volume, blood loss flow rate or other relevant parameters may also automatically be monitored by an alarm device, for example integrated in control unit 16, that issues an alarm signal when the blood loss volume and/or blood loss flow rate and/or other parameters exceeds a threshold value. The alarm signal may be any suitable signal such as a visible or an audible signal. It is remarked that instead of control unit 16, any other (processing) unit or device may also be used to determine/calculate the blood loss volume or blood loss flow rate based on input from the hematocrit sensor.

Referring to **Fig. 8****,** there may be notifications and/or information (e.g., said alarms) providable to the medical staff as shown in the table of **Fig. 8****.** Preferably, there may be notifications and/or information (e.g., said alarms) if the trend of column exceeds the values as indicated in the table of **Fig. 8****.**

In view of the embodiment of **Fig. 7****,** it should be understood that any element described therewith may also be integrated, used and/or included in connection with the embodiments of shown in **Figs. 1** **and** **6** (either alone or in any combination), such as the sensor units in connection with the control unit being correspondingly configured, the display device, the alarm device etc.

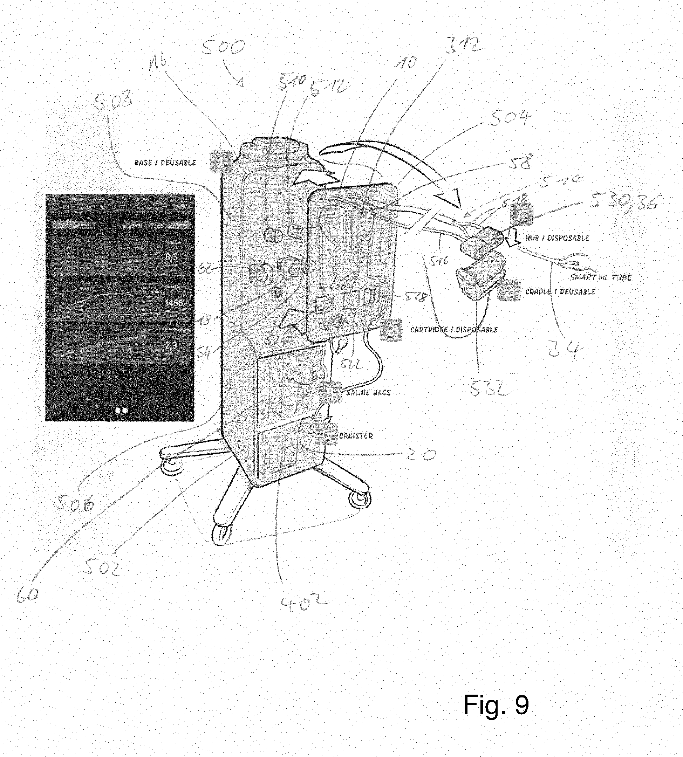

Now turning to **Fig. 9****,** a flushing system 500 according to another embodiment is schematically shown. The flushing system 500 is substantially configured as the flushing systems 1, 300, 400 described above. Hence, only the differences shall be described in the following.

It should be understood that, in view of the embodiment of **Fig. 9****,** any element described therewith may also be integrated, used and/or included in connection with the embodiments of shown in figures 1 to 8, and vice versa.

The flushing system 500 substantially comprises a base construction 502 and a frame construction 504. It may also be conceivable that a cartridge may be used instead of the frame construction 504.

The frame construction 504 is formed as a disposable frame construction.

The base construction 502 is formed as a mobile base construction being movable over the ground.

The base construction 502 comprises a bottom portion 506 and a top portion 508.

The infusion liquid container 60, here, as an example, four saline bags, and the effusion liquid container 20 are disposed and arranged in the bottom portion 506.

Further, the suction device 402 is also disposed and arranged in the bottom portion 506.

The control unit 16, the first pump device 18, the second pump device 62 and the hematocrit sensor 54 are disposed and arranged in and/or on the top portion 508.

Further, a first valve 510 and a second valve 512 are disposed and arranged in and/or on the top portion 508.

The top portion 508 is configured to hold and/or fix the frame construction 504 onto the base construction 502 in a removable and/or exchangeable manner (depicted by corresponding arrows in Fig. 9). It may be conceivable that the frame construction 504 may be mountable and/or fixable to the rest of the system, here, the base construction 502, by mechanical means, e.g., by clamping means and/or locking means and/or latching means (not shown in the figure), as known in the art.

The buffer container 10 and the second buffer container 312 are configured as a part of the frame construction 504.

In other words, the buffer container 10 and the second buffer container 312 are disposed on and are integrated in the frame construction 504.

As can be seen in **Fig. 9****,** the frame construction 504 holds and/or comprises one or more connecting lines, e.g., connecting tubes 514.

In particular, the integrated liquid interface device 36 is assigned to the frame construction 504 and is connected thereto by said connecting tubes 514, wherein one infusion connecting tube 516 is assigned to infusion liquid and two effusion connecting tubes 518 are assigned to effusion liquid.

One effusion connecting tube 518 is connected to the buffer container 10 and the other effusion connecting tube 518 is connected to the second buffer container 312.

The infusion connecting tube 516 connects the infusion liquid container 60 with the integrated liquid interface device 36 via the frame construction 504.

Downstream form the buffer containers 10, 312 and originating therefrom, further effusion connecting tubes 520 are merged in one still further effusion connecting tube 522 by means of a fluid connector piece, e.g., a Y-shaped connector.

Said still further effusion connecting tube 522 is then/further downstream merged with a connecting tube originating from the second buffer outlet 58 of the buffer containers 10, 312 by means of another fluid connector piece, e.g., another Y-shaped connector, before terminating in and/or at the effusion liquid container 20.

The frame construction 504 at least comprises a first, a second and a third access element 524, 526, 528.

The first access element 524 is configured for engaging with the second pump device 62 such that the infusion connecting tube 516 is engageable with the second pump device 62 for performing a peristaltic pumping action.

The second access element 526 is configured for engaging with the first pump device 18 such that the still further effusion connecting tube 522 is engageable with the first pump device 18 for performing a peristaltic pumping action.

The third access element 528 is configured for engaging with the hematocrit sensor 54 such that the still further effusion connecting tube 522 is engageable with the hematocrit sensor 54 for performing hematocrit measurements.

In the present exemplary embodiment, as can be seen in **Fig. 9****,** a single connecting line 522 (here: the still further effusion connecting tube 522) is used and, hence, a part of the single connecting line 522 (here: the still further effusion connecting tube 522) forms the above-described measurement lumen 116, e.g., the part of the single connecting line 522 (here: the still further effusion connecting tube 522) being engageable with and/or disposable within the hematocrit sensor 54 through the third access element 528.

The still further effusion connecting tube 522 is engageable with the hematocrit sensor 54 further downstream with respect to the first pump device 18.

The frame construction 504 also comprises two further recesses disposed between the buffer containers 10, 312 and the fluid connector piece merging the further effusion connecting tubes 520.

Said further recesses are configured for respectively engaging the first valve 510 and the second valve 512 such that the first valve 510 is engageable with the further effusion connecting tube 520 from the buffer container 10 and such that the second valve 512 is engageable with the further effusion connecting tube 520 from the second buffer container 312.

In particular, switching between the buffer containers 10, 312 is thereby facilitated, e.g., for focusing the measurements of the hematocrit sensor 54 to only one effusion liquid of the buffer container 10 or the second buffer container 312, which, e.g., may in turn allow measurement switching between effusion liquid from the pericardial or the pleural cavity PL, PLC.

As can be seen in **Fig. 9****,** the integrated liquid interface device 36 is formed as a hub device 530 for connecting to the integrated inflow and/or outflow drain device 34.

The hub device 530 combines two or more effusion liquid inlets, e.g., being respectively assignable to the wound and/or the body cavity, in particular the pericardial and/or pleural cavity PC, PLC.

The system 500 further comprises a hub receiving device 532 for holding the hub device 530.

The hub receiving device 532 is formed as a cradle device.

The hub receiving device 532 includes and/or houses the temperature sensor 412 of the system 500, e.g., to measure a/the temperature of infusion liquid, e.g., for controlling the inflow by the control unit 16.

Further, the hub receiving device 532 includes and/or houses the heater device 64 of the system 500, e.g., to heat the infusion liquid being intended to be guided to the wound and/or the body cavity, in particular the pericardial and/or pleural cavity PC, PLC, to a desired infusion liquid temperature.

It should be understood that the flushing system 500 substantially operates in the same way as the flushing systems 1, 200, 300, 400 described further above.

The flushing system 500 provides a compact and mobile solution for flushing a wound and/or a body cavity, which may allow a flexible use in a clinical routine.

In particular, the flushing system 500 provides a compact and/or cost effective solution comprising only one pump device 18 for effusion liquid and one hematocrit sensor 54, while still being able to measure effusion liquid from different origins, e.g., the pericardial and the pleural cavity, individually.

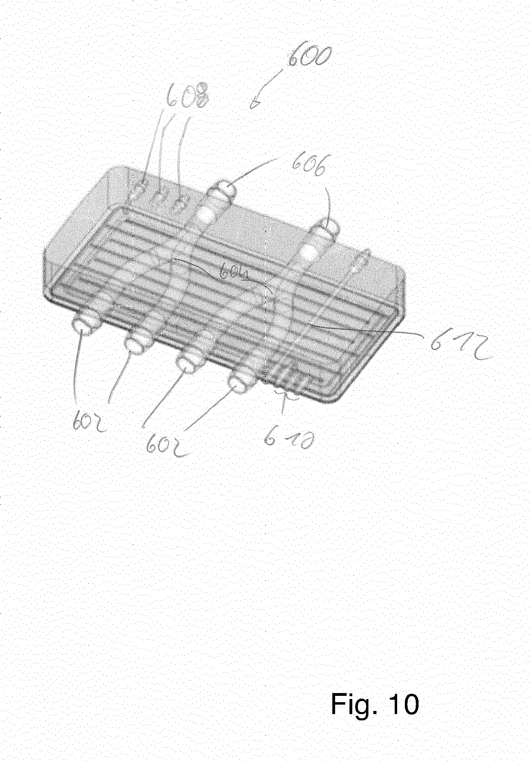

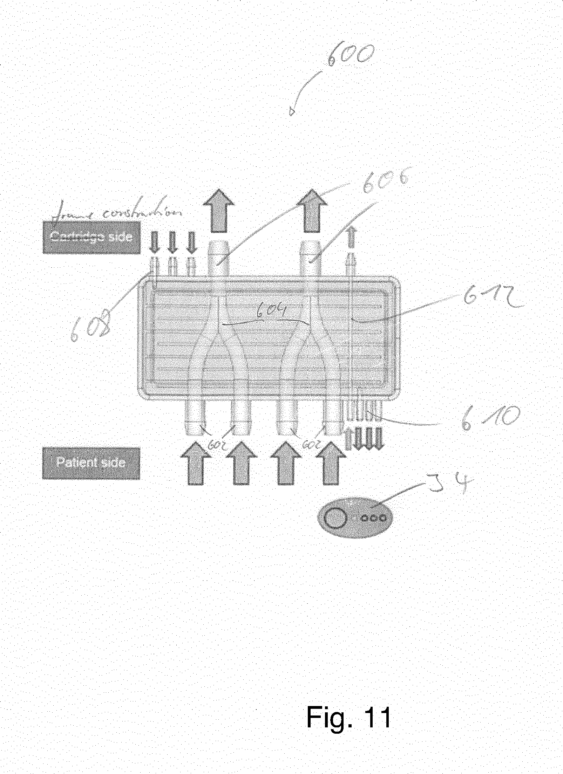

Now turning to **Figs. 10** **and** **11****,** a component, in particular a hub device 600, of a flushing system according to another embodiment of the present invention is schematically shown. The hub device 600 could be used, for example, in the flushing system 300 of Fig. 6.

The hub device 600 is substantially cuboid-shaped. However, any other shape may be conceivable.

The hub device 600 combines four effusion liquid inlets 602 being disposed on one side, e.g., the patient side, of the hub device 600.

The hub device 600 comprises two fluid connector pieces 604 being disposed in the hub device 600 and being formed as Y-shaped connectors for respectively combining two effusion liquid streams receivable via the corresponding effusion liquid inlets 604.

The hub device 600 comprises two outlets 606 for letting out the merged effusion liquid streams.

The outlets 606 are disposed on the opposite side of the hub device 600, e.g. the system side, in particular, the side of the frame construction 504, (with respect to the one side, e.g., the patient side).

Further, the hub device 600 comprises one or more infusion inlets 608 on the system side for receiving infusion liquid and one or more infusion outlets 610 on the patient side for letting out infusion liquid.

Additionally, the hub device 600 comprises a measurement channel 612, e.g., for air, having an inlet on the patient side and an outlet of the system side.

It should be understood that the patient side of the hub device 600 is configured for connecting to the integrated inflow and/or outflow drain device 34 and that, accordingly, the integrated inflow and/or outflow drain device 34 is correspondingly configured to connecting to the patient side of the hub device 600, at least partly as shown in Fig. 11 (see cross section of the integrated inflow and/or outflow drain device 34).

It should be understood that the system, in which the hub device 600 is usable, comprises a tube arrangement corresponding to the system side of the hub device 600.

It should be further understood that the arrows depicted in Fig. 11 indicate the respective stream direction of the corresponding fluids to be guided therethrough.

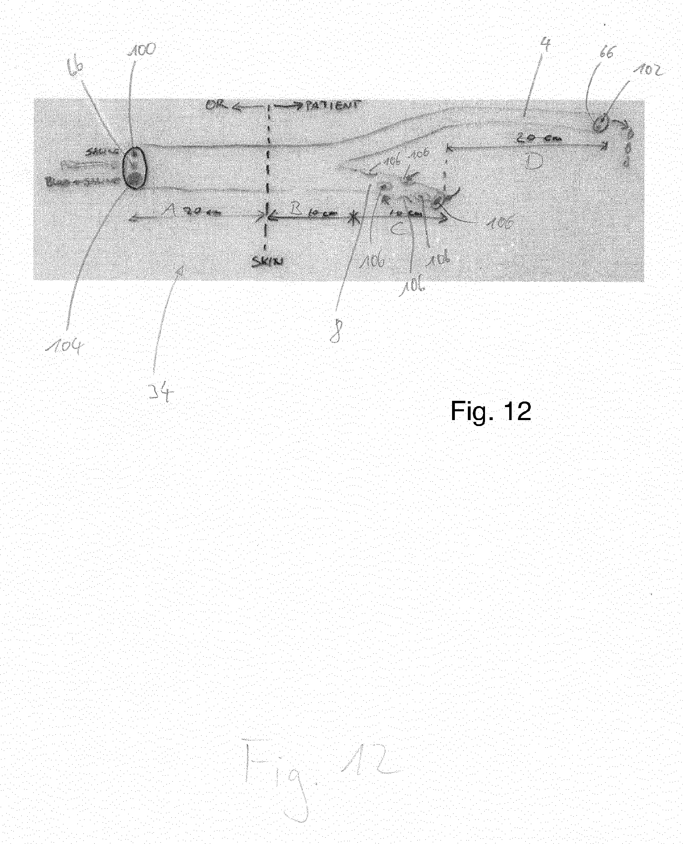

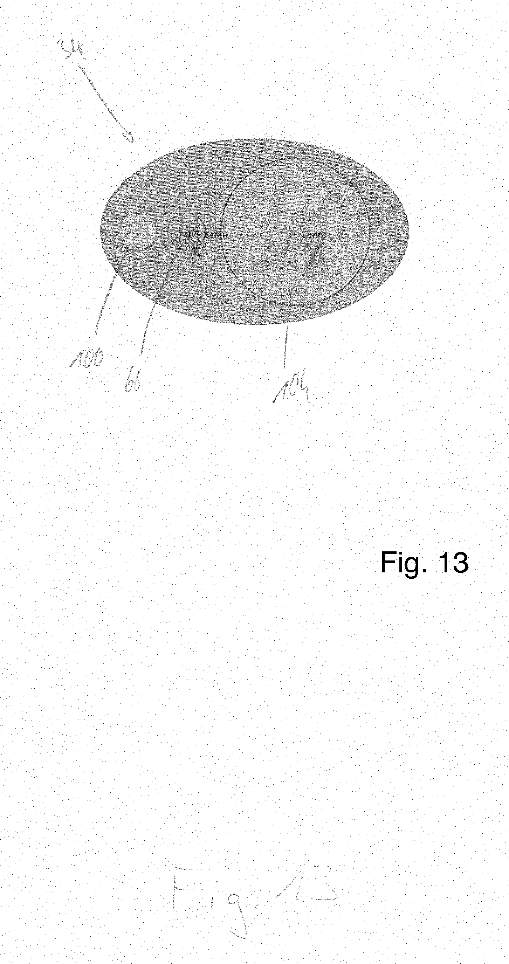

Turning to **Figs. 12** **and** **13****,** an integrated inflow and/or outflow drain device 34 for a flushing system 1, 200, 300, 500 according to an embodiment of the present invention is schematically shown. For example, the integrated inflow and/or outflow drain device 34 shown in **Figs. 12** **and** **13** may, for example, be the integrated inflow and/or outflow drain device 34 as described in connection with Fig. 1 and/or 6.

The integrated inflow and/or outflow drain device 34 of **Figs. 12** **and** **13** is substantially configured as the integrated inflow and/or outflow drain device 34 of **Figs. 2A** **and** **2B** and/or **Figs. 3A** **and** **3B** described above. Hence, only the differences shall be described in the following.

As exemplarily indicated in **Fig. 12****,** the integrated inflow and/or outflow drain device 34 is insertable into a patient (cf. dashed line may indicate a skin of a patient in case of the integrated inflow and/or outflow drain device 34 being inserted into a patient, e.g., during / for a flushing treatment). Accordingly, the integrated inflow and/or outflow drain device 34 may be subdivided into an operation room side (here, left in Fig. 12) and a patient side (here, right in Fig. 12) being intended for insertion into a patient.

The integrated inflow and/or outflow drain device 34 has an extension of distance A originating from the effusion liquid inlet 6 and, accordingly, the integrated liquid interface device 36, e.g., a or the hub device 530, 600, and extending to the intended transition between the before-mentioned sides.

Then, the integrated inflow and/or outflow drain device 34 is intended to be inserted into a patient and has an extension of distance B+C+D for being inserted into a patient.

In particular, after distance B the integrated inflow and/or outflow drain device 34 is subdivided into two branches, wherein one branch is assigned to infusion liquid and comprises the infusion liquid lumen 100 and the other branch is assigned to effusion liquid and comprises the effusion liquid lumen 104.

The branch assigned to infusion liquid has an extension of distance C+D.

The branch assigned to effusion liquid has an extension of distance C.

The effusion liquid lumen 104 of the second tube 8 has a plurality of effusion liquid openings 106 for receiving the effusion liquid from the pericardial cavity PC, in particular, in the present case, six effusion liquid openings 106.

The plurality of effusion liquid openings 106 are distributed and spaced to each other over the distance C along and around the second tube 8.

As can be seen in **Fig. 12****,** the first tube arrangement 4 is longer than the second tube 8.

In this respect, the first tube arrangement 4 is dimensioned with respect to the second tube 8 such as to allow that the effusion liquid openings 106 are spaced from the infusion liquid opening 102.

The distance D is dimensioned such as to allow that the effusion liquid openings 106 and the infusion liquid openings 102 are arrangeable at different levels with respect to the gravitational direction.

As an example, distance A may be approximately twice as long as distance B, distance B may be approximately as long as distance C, and distance D may be approximately as long as distance A. For example, distance A may be approximately 10 cm. However, it should be understood that any other suitable relation between the distances may be conceivable and/or distance A may have any other suitable value.

Further, as can be seen in **Figs. 12** **and** **13****,** the pressure sensor 66 is integrated in the integrated inflow and/or outflow drain device 34 in form of a measurement channel, e.g., measurement channel 612, for measuring the pressure by means of air. This may provide an alternative to pressure measurement in the infusion inflow and this may provide a solution with no pressure noise from the pump devices, no risk of height difference effect in the fluid column, and with a low-cost implementation. The exit point of the measurement channel (in the pericardium) for pressure measurement may be next to and/or close to the infusion inflow openings (in the pericardium), so the infusion liquid keeps the measurement channel free from clots.

Referring to **Fig. 13****,** a cross-sectional view of the integrated inflow and/or outflow drain device 34 is schematically shown, wherein the cross-section is taken within distance A.

The cross-sectional outline of the integrated inflow and/or outflow drain device 34 is substantially oval-shaped.

The pressure sensor 66 in form of the measurement channel is disposed between the infusion liquid lumen 100 and the effusion liquid lumen 102.

The diameter of the infusion liquid lumen 100 is smaller than the diameter of the effusion liquid lumen 102.

For example, the relation between said diameters may be approximately 1 to 3 or 1 to 4.

In this respect, the diameter of the infusion liquid lumen 100 may be approximately 1,5 mm to approximately 2 mm, and the diameter of the effusion liquid lumen 102 may be approximately 6 mm. However, any other suitable diameters may be conceivable.

Further, as can be seen in **Fig. 13****,** the dashed line indicates where the respective branches are separating, e.g., may be separated during manufacturing of the integrated inflow and/or outflow drain device 34.

The integrated inflow and/or outflow drain device 34 may have a silicone base. In other words, the integrated inflow and/or outflow drain device 34 may be manufactured from a silicone material, in particular, a medical grade silicone material.

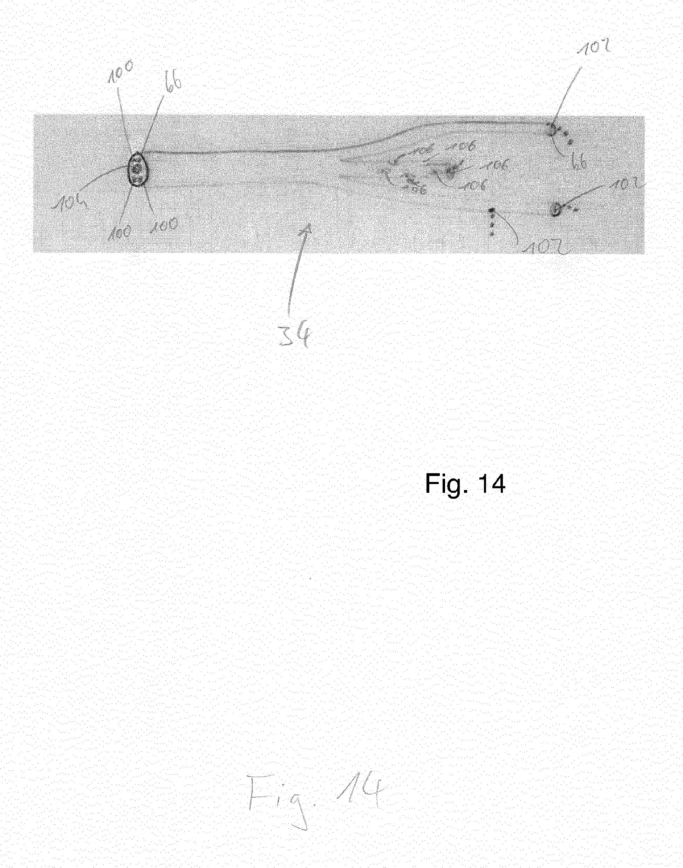

Turning to **Fig. 14****,** an integrated inflow and/or outflow drain device 34 for a flushing system 1, 200, 300, 500 according to an embodiment of the present invention is schematically shown. For example, the integrated inflow and/or outflow drain device 34 shown in **Fig. 14** may, for example, be the integrated inflow and/or outflow drain device 34 as described in connection with Fig. 1 and/or 6.

The integrated inflow and/or outflow drain device 34 of **Fig. 14** is substantially configured as the integrated inflow and/or outflow drain device 34 of **Figs. 12** **and** **13** described above. Hence, only the differences shall be described in the following.

As can be seen in **Fig. 14****,** the integrated inflow and/or outflow drain device 34 comprises three infusion liquid lumen 100.

Further, the integrated inflow and/or outflow drain device 34 comprises two branches assigned to infusion liquid, wherein one of these branches comprises one infusion liquid lumen 100 and the other branch comprises two infusion liquid lumen 100.

Further, the branch comprising the two infusion liquid lumen 100 respectively comprises corresponding infusion liquid openings 102 being transversely oriented to each other.

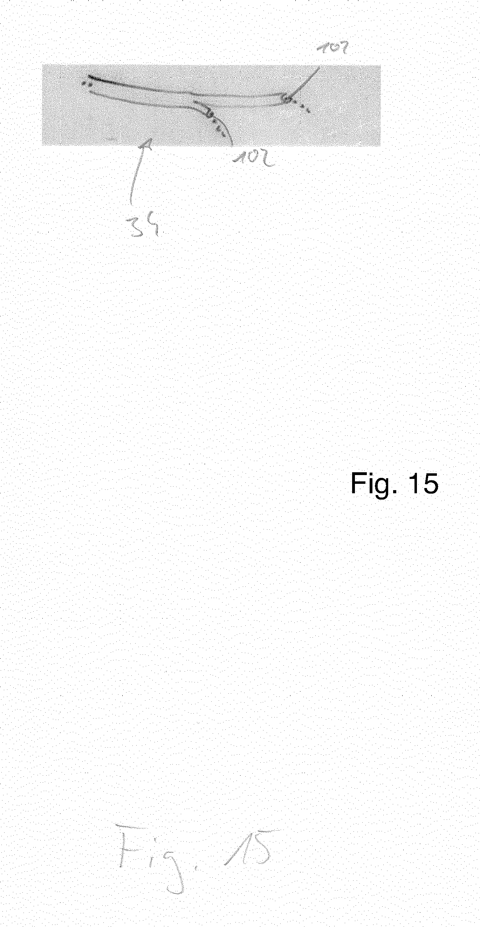

Turning to **Fig. 15****,** a portion of an integrated inflow and/or outflow drain device 34 for a flushing system 1, 200, 300, 500 according to an embodiment of the present invention is schematically shown.

Said portion may be an alteration of the integrated inflow and/or outflow drain device 34 of **Figs. 12****,** **13** **and/or 14.**

In particular, said portion is a branch assigned to infusion liquid, which in turn is again sub-branched into two sub-branches.

Each sub-branch houses one infusion liquid lumen 100 with a respective infusion liquid opening 102 at its end.

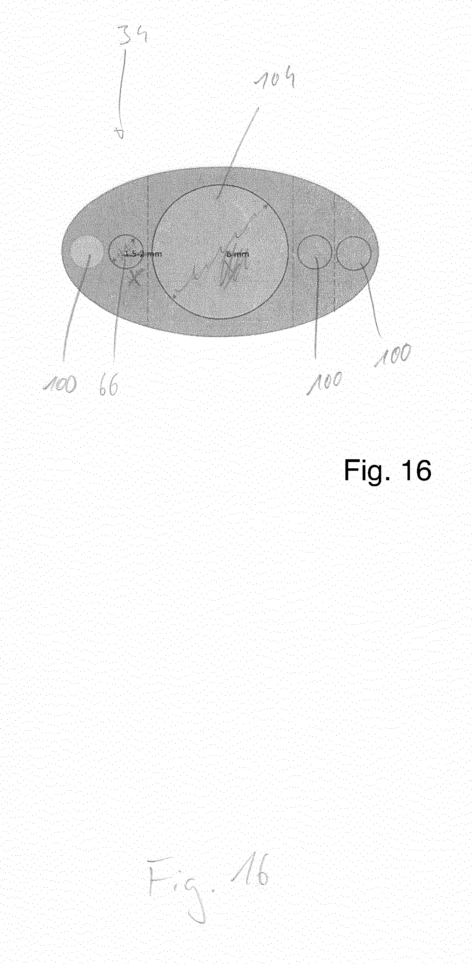

Turning to **Fig. 16****,** a cross-section of an integrated inflow and/or outflow drain device 34 for a flushing system 1, 200, 300, 500 according to an embodiment of the present invention is schematically shown.

Said cross-section may be an alteration of the integrated inflow and/or outflow drain device 34 of **Figs. 12****,** **13****,** **14** **and/or 15.**

In particular, said cross-section is substantially configured as the cross-section of **Fig. 13** described above. Hence, only the differences shall be described in the following.

The effusion liquid lumen 104 is disposed between a first pair of the pressure sensor 66 in form of a/the measurement channel and one infusion liquid lumen 100 and a second pair of two infusion liquid lumen 100.

The first pair, the effusion liquid lumen 104 and each lumen 100 of the second pair are arranged such as to be branched from each other, e.g., as indicated by the dashed lines.

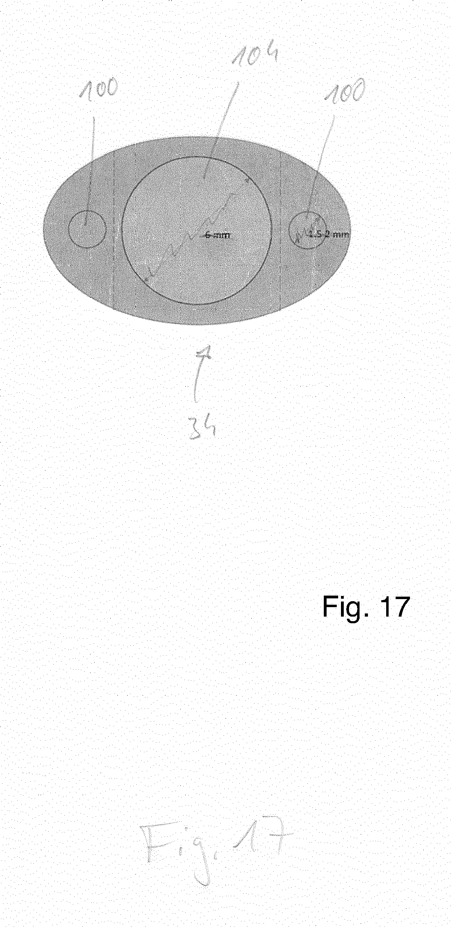

Turning to **Fig. 17****,** a cross-section of an integrated inflow and/or outflow drain device 34 for a flushing system 1, 200, 300, 500according to an embodiment of the present invention is schematically shown.

Said cross-section may be an alteration of the integrated inflow and/or outflow drain device 34 of **Figs. 12****,** **13****,** **14****,** **15** **and/or 16.**

In particular, said cross-section is substantially configured as the cross-section of **Fig. 13** described above. Hence, only the differences shall be described in the following.

The effusion liquid lumen 104 is disposed between two infusion liquid lumen 100.

The effusion liquid lumen 104 and each infusion liquid lumen 100100 are arranged such as to be branched from each other, e.g., as indicated by the dashed lines.

Further, no measurement channel is formed in the integrated inflow and/or outflow drain device 34.

It should be understood that the hub device 530, 600 described herein may be configured to be engageable and/or connectable with any of the integrated inflow and/or outflow drain devices 34 described herein, such as the integrated inflow and/or outflow drain devices 34 of **Figs. 12****,** **13****,** **14****,** **15****,** **16** **and/or 17.**

### Reference Signs

- 1: flushing system
- 2: infusion liquid outlet
- 4: first tube arrangement
- 6: effusion liquid inlet, first effusion liquid inlet
- 8: second tube
- 10: buffer container, first buffer container
- 12: buffer inlet
- 14: first buffer outlet
- 16: control unit
- 18: first pump device
- 20: effusion liquid container
- 22: clot trap
- 24: top end of buffer container
- 26: bottom end of buffer container
- 28: top wall of buffer container
- 30: side wall of buffer container
- 32: bottom wall of buffer container
- 34: integrated inflow and/or outflow drain device
- 36: integrated liquid interface device
- 38: further effusion liquid inlet, second effusion liquid inlet
- 40: further tube, third tube
- 42: fluid connector piece
- 44: sieve structure
- 46: base of clot trap
- 48: apex of clot trap
- 50: first level sensor
- 52: second level sensor
- 54: hematocrit sensor
- 56: intensive care unit (ICU) vacuum wall connector
- 58: second buffer outlet
- 60: infusion liquid container
- 62: second pump device
- 64: heater device
- 66: pressure sensor

- 100: infusion liquid lumen
- 100a: first infusion liquid lumen
- 100b: second infusion liquid lumen
- 100c: third infusion liquid lumen
- 102: infusion liquid opening
- 102a: first infusion liquid opening
- 102b: second infusion liquid opening
- 102c: third infusion liquid opening
- 104: effusion liquid lumen
- 106: effusion liquid opening
- 106a: first effusion liquid opening
- 106b: second effusion liquid opening
- 106c: third effusion liquid opening
- 108: temperature sensor
- 110: retractable sheath

- 300: flushing system
- 302: third effusion liquid inlet
- 304: fourth tube
- 306: fourth effusion liquid inlet
- 308: fifth tube
- 310: fluid connector piece
- 312: further buffer container, second buffer container
- 314: further pump device, third pump device
- 316: further hematocrit sensor, second hematocrit sensor

- 400: flushing system
- 402: suction device
- 404: weight sensor
- 406: flow rate sensor
- 408: weight sensor
- 410: flow rate sensor
- 412: temperature sensor
- 414: display device

- 500: flushing system
- 502: base construction
- 504: frame construction
- 506: bottom portion
- 508: top portion
- 510: first valve
- 512: second valve
- 514: connecting tube
- 516: infusion connecting tube
- 518: effusion connecting tube
- 520: further effusion connecting tube
- 522: still further effusion connecting tube
- 524: first access element
- 526: second access element
- 528: third access element
- 530: hub device
- 532: hub receiving device

- 600: hub device
- 602: effusion liquid inlet
- 604: connector piece
- 606: outlet
- 608: infusion inlet
- 610: infusion outlet
- 612: measurement channel
- PC: pericardial cavity
- PLC: pleural cavity

## Claims

1. A flushing system (1, 300, 400, 500) configured to flush a wound and/or a body cavity, in particular the pericardial cavity (PC), of a patient, wherein the system (1, 300, 400, 500) comprises:
- an infusion liquid outlet (2) to connect to a first tube arrangement (4) having one or more infusion liquid lumen (100, 100a, 100b, 100b) to guide a flow of infusion liquid from the system (1, 300, 400, 500) to the wound and/or the body cavity, in particular the pericardial cavity (PC),
- an effusion liquid inlet (6) to connect to a second tube (8) having an effusion liquid lumen (104) to guide the effusion liquid flow from the wound and/or the body cavity, in particular the pericardial cavity (PC), to the system (1, 300, 400, 500),
- a flow rate control system to control the flow rate of the infusion liquid flow at the infusion liquid outlet (2), wherein the flow rate control system comprises:
▪ a control unit (16) to provide one or more control signals, and
▪ a pump device (62) to pump infusion liquid to the infusion liquid outlet (2) at an infusion liquid flow rate, wherein the infusion liquid flow rate is adjustable by the control signals of the control unit (16),
wherein each infusion liquid lumen (100, 100a, 100b, 100b) of the first tube arrangement (4) has a t least one infusion liquid opening (102, 102a, 102b, 102b) for leading the infusion liquid into the wound and/or the body cavity, in particular the pericardial cavity (PC), wherein the infusion liquid openings (102, 102a, 102b, 102b) are arranged along the first tube arrangement (4) and are spaced from one another.

2. The system (1, 300, 400, 500) of claim 1,
**characterized in that**
the first tube arrangement (4) includes a retractable sheath (110) being retractable between a first position and a second position via at least one intermediate position, along the first tube arrangement (4), wherein, in the first position, the infusion liquid openings (102, 102a, 102b, 102b) are covered by the sheath (110), in the second position, the infusion liquid openings (102, 102a, 102b, 102b) are not covered by the sheath (110), and, in the intermediate position, some of the infusion liquid openings (102, 102a, 102b, 102b) are covered by the sheath (110).

3. The system (1, 300, 400, 500) of claim 1 or 2,
**characterized in that**
the first tube arrangement (4) is arrangeable in a substantially horizontal orientation for leading the infusion liquid into the wound and/or the body cavity, in particular the pericardial cavity (PC).

4. The system (1, 300, 400, 500) of any of the preceding claims,
**characterized in that**
the effusion liquid lumen (104) of the second tube (8) has at least one effusion liquid opening (106, 106a, 106b, 106c) for receiving the effusion liquid from the wound and/or the body cavity, in particular the pericardial cavity (PC).

5. The system (1, 300, 400, 500) of claim 4,
**characterized in that**
the first tube arrangement (4) and the second tube (8) are configured to be arranged such that the effusion liquid openings (106, 106a, 106b, 106c) and the infusion liquid openings (102, 102a, 102b, 102c) are disposed at different levels with respect to the gravitational direction for allowing the infusion liquid to either work with or against gravity, when applied in the wound and/or the body cavity, in particular the pericardial cavity (PC).

6. The system (1, 300, 400, 500) of any of the preceding claims,
**characterized in that**
the first tube arrangement (4) and the second tube (8) are combined in an integrated inflow and/or outflow drain device (34).

7. The system (1, 300, 400, 500) of claim 6,
**characterized in that**
the integrated first tube arrangement (4) of the inflow and/or outflow drain device (34) is longer than the integrated second tube (8), optionally, wherein the integrated first tube arrangement (4) has a length such as to allow that the effusion liquid openings (106, 106a, 106b, 106c) and the infusion liquid openings (102, 102a, 102b, 102c) are arranged or arrangeable at different levels with respect to the gravitational direction.

8. The system (1, 300, 400, 500) of any of the preceding claims,
**characterized in that**
the system (1, 300, 400, 500) further comprises one or more effusion liquid containers (20) to receive effusion liquid from the wound and/or the body cavity, in particular the pericardial cavity (PC).

9. The system (1, 300, 400, 500) of any of the preceding claims,
**characterized in that**
the system (1, 300, 400, 500) further comprises a hematocrit sensor (54) configured to measure the hematocrit value of the effusion liquid for obtaining a blood loss volume and/or a blood loss flow rate from the wound and/or the body cavity, in particular the pericardial cavity (PC).

10. The system (1, 300, 400, 500) of claim 9,
**characterized in that**
the hematocrit sensor (54) is disposed between the effusion liquid inlet (6) and one or more effusion liquid containers (20) for conducting measurements in the effusion liquid.

11. The system (1, 300, 400, 500) of any of the preceding claims,
**characterized in that**
the system (1, 300, 400, 500) further comprises a pressure sensor (66) being positioned inside or in connection with the first tube arrangement (4), the second tube (8) or the wound and/or the body cavity, in particular the pericardial cavity (PC), and being configured for obtaining the pressure in the wound and/or the body cavity, in particular the pericardial cavity (PC).

12. The system (1, 300, 400, 500) of any of the preceding claims,
**characterized in that**
the system (1, 300, 400, 500) further comprises a heater device (64) configured to heat the infusion liquid being intended to be guided to the wound and/or the body cavity, in particular the pericardial cavity (PC), to a desired infusion liquid temperature, optionally, wherein the desired infusion liquid temperature is based on a temperature of the wound and/or the body cavity, in particular the pericardial cavity (PC), in particular a desired temperature of the wound and/or the body cavity, in particular the pericardial cavity (PC), and/or a general body temperature.

13. The system (300, 500) of any of the preceding claims,
**characterized in that**
the system (300, 500) further comprises a further effusion liquid inlet (302) to connect to a third tube (304) having an effusion liquid lumen to guide the effusion liquid flow from a further wound and/or a further body cavity, in particular the pleural cavity (PLC), to the system (300), and
optionally, the system (300) further comprises a further hematocrit sensor (316) configured to measure the hematocrit value of the effusion liquid for obtaining a blood loss volume and/or a blood loss flow rate from the further wound and/or the further body cavity, in particular the pleural cavity (PLC), optionally, wherein the further hematocrit sensor (316) is disposed between the further effusion liquid inlet (302) and one or more effusion liquid containers (20) for conducting measurements in the effusion liquid, and/or wherein
the system further comprises at least one weight sensor (404), which is to measure a change in weight of the infusion liquid in at least a part and/or component of the system, especially in infusion liquid container (60) of the system.

14. Tube arrangement (4) for a flushing system (1, 300, 400, 500) configured to flush a wound and/or a body cavity, in particular the pericardial cavity (PC), of a patient, in particular for a flushing system of any of the preceding claims, wherein the tube arrangement (4) comprises:
one or more infusion liquid lumen (100, 100a, 100b, 100b) configured to guide a flow of infusion liquid from the flushing system (1, 300, 400) to the wound and/or the body cavity, in particular the pericardial cavity (PC), when arranged and/or operated in the flushing system (1, 300, 400, 500),
wherein each infusion liquid lumen (100, 100a, 100b, 100b) of the first tube arrangement (4) has at least one infusion liquid opening (102, 102a, 102b, 102b) for leading the infusion liquid into the wound and/or the body cavity, in particular the pericardial cavity (PC), wherein the infusion liquid openings (102, 102a, 102b, 102b) are arranged along the first tube arrangement (4) and are spaced from one another.

15. Tube arrangement (4) of claim 14,
**characterized in that**
the first tube arrangement (4) includes a retractable sheath (110) being retractable between a first position and a second position via at least one intermediate position, along the first tube arrangement (4), wherein, in the first position, the infusion liquid openings (102, 102a, 102b, 102b) are covered by the sheath (110), in the second position, the infusion liquid openings (102, 102a, 102b, 102b) are not covered by the sheath (110), and, in the intermediate position, some of the infusion liquid openings (102, 102a, 102b, 102b) are covered by the sheath (110).
